# FASCICULE DE BREVET EUROPEEN

(11) **EP 3 576 713 B1**
(45) Date de publication et mention de la délivrance du brevet: **09.12.2020**
(21) Numéro de dépôt: 18705446.5
(22) Date de dépôt: 01.02.2018
(51) Int. Cl.: A61K 8/37, A61Q 1/06, A61Q 19/00, A61K 8/891, A61K 8/894, A61K 8/92, A61K 8/02, A61K 8/06

(54) **ÉMULSION COSMÉTIQUE EAU-DANS-HUILE SOLIDE**
FESTE KOSMETISCHE WASSER-IN-ÖL-EMULSION
SOLID WATER-IN-OIL COSMETIC EMULSION

(30) Priorité: 02.02.2017 FR 1750887
(43) Date de publication de la demande: 11.12.2019
(73) Titulaire: Chanel Parfums Beauté, 92200 Neuilly-sur-Seine (FR)
(72) Inventeur: RAVNI, Clémence, 75019 Paris (FR); DE CLERMONT-GALLERANDE, Hélène, 94300 Vincennes (FR); YVERNOGEAU, Martine, 92300 Levallois Perret (FR); PICHOUTOU, Olivia, Pantin Cedex, 93694 (FR)
(74) Mandataire: Plasseraud IP
(86) Numéro de dépôt international: PCT/FR2018/050237
(87) Numéro de publication internationale: WO 2018/142076

(56) Documents cités:
- EP-A1- 1 741 422
- FR-A1- 2 860 143
- FR-A1- 2 909 874
- FR-A1- 2 967 908
- FR-A1- 2 985 180
- US-A1- 2015 202 141
- WACKER-BELSIL: "Wacker-Belsil TMS 803", INTERNET CITATION, 1 février 2006 (2006-02-01), pages 1-3, XP002638961, Extrait de l'Internet: URL:http://products.brenntagspecialties.co m/wacker/TMS_803.pdf [extrait le 2011-05-25]
- Kahlwax: "Technical Data Sheet Kahlresin 6723 - Shorea Robusta Resin + Octyldodecanol", , 8 avril 2016 (2016-04-08), page 1, XP055457909, Extrait de l'Internet: URL:https://www.ulprospector.com/documents /1501021.pdf?bs=33069&b=703236&st=20&r=eu& ind=personalcare [extrait le 2018-03-09]

## Description

La présente invention a pour objet une émulsion eau-dans-huile solide, utilisable dans le domaine cosmétique, qui se présente de préférence sous la forme d'un stick. L'invention a également pour objet un procédé de maquillage et/ou de soin non thérapeutique de la peau ou des lèvres d'êtres humains, comprenant l'application sur la peau ou les lèvres de ladite composition, de préférence sous forme de stick.

Les compositions de maquillage et/ou de soin sont couramment employées pour apporter un aspect esthétique lors de l'application sur les lèvres ou la peau, cet effet devant bien entendu perdurer au cours du temps. Elles doivent notamment résister aux différents facteurs extérieurs susceptibles de modifier leur effet esthétique, comme par exemple la salive ou la sueur. En particulier, les produits cosmétiques, et notamment les rouges à lèvres, ne doivent pas migrer ou filer dans les rides ou ridules, ou transférer sur un support. Ils doivent également être agréables à appliquer sur les lèvres ou la peau et leur dépôt doit procurer une sensation de confort à l'utilisatrice, tout en conservant des propriétés esthétiques satisfaisantes. Il est fait référence au document FR 2 985 180 décrivant une émulsion cosmétique eau-dans-huile pour le maquillage.

Les documents FR2873018, EP374332 et WO1999/47111 décrivent tous une émulsion cosmétique eau-dans-huile solide pour une application au maquillage et au soin de la peau. Cependant ces émulsions comportent toutes une quantité d'huile volatile assez importante, ce qui peut présenter certains inconvénients.

Les huiles volatiles, et notamment les huiles siliconées volatiles sont largement utilisées depuis les années 1995 dans la formulation des rouges à lèvres, dans le but de remplacer les huiles végétales et les paraffines liquides classiques habituellement utilisées. En effet, l'utilisation d'huile de silicone volatile améliore la propriété de « non-transférabilité » notamment recherchée pour les rouges à lèvres, à savoir l'absence de trace de rouge sur un support qui rentre en contact avec les lèvres (un verre, une tasse, la peau, un vêtement ...).

Cependant, les rouges à lèvres à base de silicone volatile sont mats, friables et présentent un confort tout à fait relatif (notamment la sensation de tiraillement due à l'évaporation des huiles volatiles). De plus l'évaporation des silicones induit un rétrécissement du diamètre du stick du rouge dans le temps. A long terme, ces rouges ne vieillissent pas très bien car une petite perte en silicone rend encore plus inconfortable et déshydratant le rouge à lèvres. De plus, la rupture du stick à la base de la cupule est alors facilitée.

Les huiles volatiles à chaîne hydrocarbonée courte, telles que l'isododécane, l'isohexadécane, sont également utilisées pour optimiser la non transférabilité du rouge à lèvres et éviter les trop grandes incompatibilités de formulation liées aux silicones volatiles.

Cependant, quelle que soit la nature des composés volatils, leur incorporation dans une formule pose des problèmes lors de la fabrication des produits. En effet, les sticks pour la peau ou les lèvres sont fabriqués à chaud pour permettre la fonte des cires. L'ajout de substances volatiles, siliconées ou minérales, se fait au dernier moment de la fabrication, à une température aussi basse que possible pour éviter une trop grande évaporation de ces solvants. En effet, l'évaporation des solvants volatils rend d'une part difficile la maîtrise de la composition finale du produit. D'autre part, si les points éclairs des solvants volatiles sont bas, en particulier inférieurs à la température de chauffe du « bulk » (à savoir le produit dans la cuve) pendant la fabrication du produit, se posent alors des problèmes de sécurité qui nécessitent l'utilisation d'un équipement anti-déflagrant pour diminuer les risques de manipulation.

Pour toutes ces raisons, les Inventeurs ont cherché à obtenir une composition cosmétique pour la peau ou les lèvres, en particulier sous forme de stick, qui ne comprend pas ou très peu d'huile volatile, mais qui présente néanmoins les propriétés habituellement recherchées pour un stick pour la peau ou les lèvres.

Un but de l'invention est de proposer une composition de maquillage et/ou de soin de la peau ou des lèvres, présentant d'excellentes propriétés de sensorialité, à savoir une sensation de confort, de fraîcheur et de fondant au moment de l'application sur la peau ou les lèvres. On entend notamment par confort, au sens de l'invention, l'absence de sensation de tiraillement de la peau ou des lèvres.

Un autre but de l'invention est d'obtenir une composition de maquillage et/ou de soin de la peau ou des lèvres, notamment sous forme de sticks, qui présente une teneur en eau importante, à savoir au moins 30% en poids par rapport au poids total de la composition, afin d'améliorer la sensation de fraîcheur et de confort lors de son application sur la peau ou les lèvres.

Un autre but de l'invention est de proposer une composition de maquillage et/ou de soin de la peau ou des lèvres, présentant une facilité d'étalement au moment de l'application sur la peau ou les lèvres et un résultat homogène une fois la composition appliquée.

Un autre but de l'invention est de proposer un stick de maquillage et/ou de soin pour la peau ou les lèvres qui présente une bonne stabilité dans le temps, à savoir qui ne rétrécit pas par rapport à sa forme initiale, qui ne se rompt pas à sa base, qui ne vieillit pas par dessèchement ou dessiccation et qui ne présente pas de phénomène d'exsudation (à savoir le relargage d'huile ou d'eau en surface).

Un autre but de l'invention est de proposer un stick pour la peau ou les lèvres qui ne pose pas de problème de sécurité lors de la fabrication dudit stick.

Encore un autre but de l'invention est de proposer un stick pour la peau ou les lèvres présentant de bonnes propriétés de stabilité, d'apparence et de sensorialité et dont la fabrication ne requiert pas des températures de chauffe trop élevées, ne dépassant pas 95°C.

Il est du mérite de la Demanderesse d'avoir observé qu'il était possible de s'affranchir de la présence d'huiles volatiles habituellement utilisées dans les compositions cosmétiques, et notamment de rouge à lèvres, de l'art antérieur.

On entend par « s'affranchir » au sens de l'invention le fait de ne pas avoir du tout d'huiles volatiles dans les compositions ou alors en quantité très faible.

En effet, la Demanderesse a observé qu'une combinaison spécifique de composés particuliers permettait avantageusement d'obtenir des compositions cosmétiques de maquillage et/ou de soin sous forme de stick pour la peau ou les lèvres, présentant l'ensemble des propriétés recherchées dans le cadre de l'invention (dépôt d'un film mat ou brillant, absence de tiraillement de la peau ou des lèvres, sensation de fraîcheur, stick qui ne rétrécit pas et ne vieillit pas prématurément, fabrication du stick non contraignante ...).

L'invention a ainsi pour objet une composition cosmétique sous la forme d'une émulsion eau-dans-huile solide caractérisée en ce qu'elle comprend :
- un mélange émulsionnant comprenant au moins un émulsionnant siliconé présent dans une quantité comprise entre 0,1% et 5% et au moins un silicone élastomère émulsionnant ledit silicone élastomère étant présent dans une quantité comprise entre 0,05% et 3%,
- au moins une huile non volatile présente dans une quantité comprise entre 30% et 50% et comprenant au moins une huile non volatile siliconée et éventuellement une seconde huile choisie parmi au moins une huile ester non volatile,
- un agent structurant de la phase huileuse comprenant une cire et/ou un gélifiant huileux,
- 30% à 50% d'eau,
- 0% à 2%, et plus préférentiellement encore 0% d'une huile siliconée volatile et/ou d'une huile hydrocarbonée volatile, et
- optionnellement un filmogène ;
les pourcentages définis ci-dessus étant des pourcentages en poids par rapport au poids total de la composition.

Par « émulsion solide », on entend selon l'invention une émulsion qui ne s'écoule pas sous son propre poids pendant 1h à 25°C. Cette émulsion solide est cependant coulable à chaud, à des températures comprises entre 55°C et 95°C afin de se présenter sous la forme d'un stick, de préférence d'un stick de maquillage et/ou de soin pour la peau ou les lèvres.

### Emulsionnant siliconé

L'émulsionnant siliconé est un polymère de siloxane comprenant :
- une chaîne latérale grasse,
- une chaîne latérale oxyéthylénée ou oxypropylénée et/ou une chaîne latérale polyéthoxylée, et éventuellement
- une chaîne latérale siliconée.

La chaîne latérale grasse de l'émulsionnant siliconé permet d'avoir une bonne comptabilité avec la phase grasse de l'émulsion eau-dans-huile.

La chaîne latérale siliconée permet d'avoir une bonne comptabilité avec l'huile siliconée non volatile. Selon un mode de réalisation de l'invention, l'émulsionnant siliconé comprend une chaîne latérale grasse et une chaîne latérale siliconée.

Plus particulièrement selon l'invention, l'émulsionnant siliconé est choisi dans le groupe comprenant :
- le composé de formule (I) : 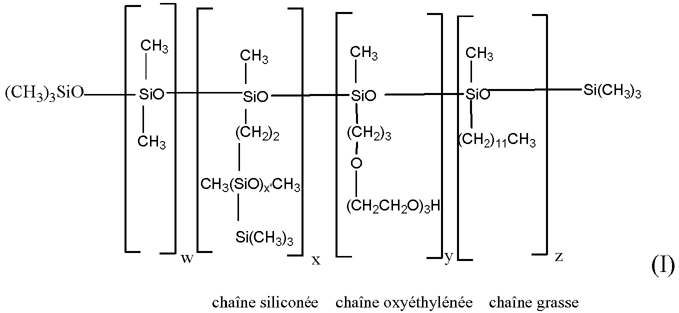 dans laquelle w est un entier compris entre 1 et 1000, x' est un entier compris entre 1 et 50, x, y et z représentent indépendamment les uns des autres un entier compris entre 1 et 100,
- le composé de formule (II) : 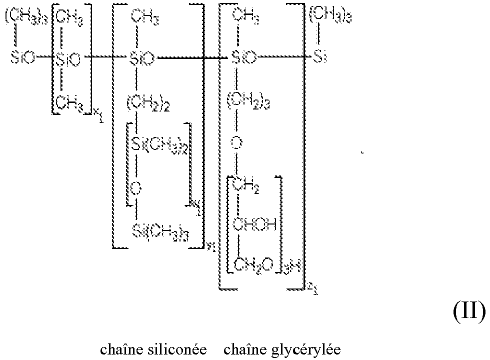 dans laquelle x₁ est un entier compris entre 1 et 1000, w₁ est un entier compris entre 1 et 50, y₁ et z₁ représentent indépendamment l'un de l'autre un entier compris entre 1 et 100,
- le composé de formule (III) : 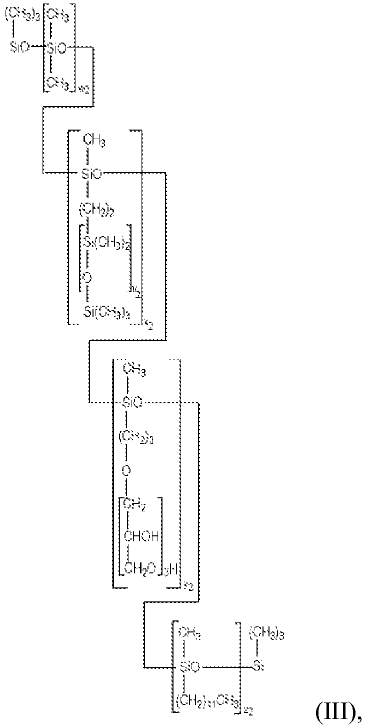 dans laquelle w₂ est un entier compris entre 1 et 1000, v₂ est un entier compris entre 1 et 50, x₂, y₂ et z₂ représentent indépendamment les uns des autres un entier compris entre 1 et 100,
   et leurs mélanges.

Selon un mode particulier de réalisation, l'émulsionnant siliconé est choisi dans le groupe comprenant les polymères de siloxane commercialisés par la société SHIN-ETSU sous les références KF6038, KF6104, KF6105, KF6106 et leurs mélanges.

Le composé KF6038, ayant pour nom INCI « Lauryl PEG-9 Polydimethylsiloxyethyl Dimethicone » répond à la formule générale (I). Ce polymère de siloxane comprend une chaîne latérale siliconée, une chaîne latérale oxyéthylénée et une chaîne latérale grasse (lauryl).

Le composé KF6104, ayant pour nom INCI « Polyglyceryl-3 Polydimethylsiloxyethyl Dimethicone », et le composé KF6106, ayant pour nom INCI « Polyglyceryl-3 Polydimethylsiloxyethyl Dimethicone » répondent à la formule générale (II). Ce polymère de siloxane comprend une chaîne latérale siliconée et une chaîne latérale glycérylée.

Le composé KF6105, ayant pour nom INCI «Lauryl Polyglyceryl-3 Polydimethylsiloxyethyl Dimethicone » répond à la formule générale (III). Ce polymère de siloxane comprend une chaîne latérale siliconée, une chaîne latérale glycérylée et une chaîne latérale grasse (lauryl).

L'émulsionnant siliconé est présent dans la composition cosmétique de l'invention en une teneur comprise entre 0,1% et 5%, de préférence comprise entre 1% et 3%, les pourcentages étant des pourcentages en poids par rapport au poids total de la composition.

### Silicone élastomère émulsionnant

L'émulsion solide eau-dans-huile comprend, en plus de l'émulsionnant siliconé tel que défini ci-dessus, un silicone élastomère émulsionnant, à savoir un organopolysiloxane réticulé élastomère, de préférence à chaîne polyhydroxylée.

L'organopolysiloxane réticulé élastomère peut être obtenu par une réaction d'addition réticulation de diorganopolysiloxane contenant au moins un hydrogène lié au silicium et de composés polyhydroxylés ou polyoxyalkylénés ayant des groupements à insaturation éthylénique, notamment en présence de catalyseur platine.

Plus particulièrement, l'organopolysiloxane réticulé élastomère est obtenu par une réaction d'addition réticulation (A) de diorganopolysiloxane contenant au moins deux hydrogènes liés chacun à un silicium et (B) de composés glycérolés ou polyoxyalkylénés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C). Le composé (A) est en particulier un organopolysiloxane ayant au moins 2 atomes d'hydrogène liés à des atomes de silicium distincts dans chaque molécule.

Le composé (A) peut présenter une structure à chaîne linéaire, à chaîne ramifiée ou une structure cyclique. Il peut présenter une viscosité à 25°C comprise entre 1 et 50 000 centistokes (cSt), notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate ou un groupe mercapto.

De préférence, ledit groupe organique est choisi parmi les groupes méthyl, phényl ou lauryl. Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane ou les copolymères diméthylsiloxane-méthylhydrogénosiloxanelaurylméthylsiloxane à terminaisons triméthylsiloxy.

Le composé (B) peut être :
- soit un composé polyglycérolé répondant à la formule (B') suivante :

   CₘH₂ₘ₋₁-O-[Gly]ₙ-CₘH₂ₘ₋₁ (B'),

   dans laquelle m est un entier compris entre 2 et 6, n est un entier compris entre 2 et 200, de préférence compris entre 2 et 100, de préférence compris entre 2 et 50, de préférence compris entre 2 et 20, de préférence compris entre 2 et 10, et plus préférentiellement encore n est un entier compris entre 2 et 5, et en particulier n est égal à 3 ; Gly désigne -CH₂-CH(OH)-CH₂-O- ou -CH₂-CH(CH₂OH)-O-,
- soit un composé polyoxyalkylène ayant deux groupes vinyliques.

Avantageusement, la somme du nombre de groupements éthyléniques par molécule du composé (B) et du nombre d'atomes d'hydrogène liés à des atomes de silicium par molécule du composé (A) est d'au moins 4.

Avantageusement, le composé (A) est ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) est de 1/1 à 20/1.

Le composé (C) est le catalyseur de la réaction de réticulation, et est choisi parmi l'acide chloroplatinique, les complexes acide chloroplatinique-oléfine, les complexes acide chloroplatinique-alkénylsiloxane, les complexes acide chloroplatinique-dicétone, le platine noir ou le platine sur support.

Le catalyseur (C) est de préférence ajouté en une quantité comprise entre 0,1 et 1000 parts en poids, de préférence comprise entre 1 et 100 parts en poids, en tant que métal platine propre pour 1000 parts en poids de la quantité totale des composés (A) et (B).

L'élastomère de silicone émulsionnant peut être véhiculé sous forme de gel dans au moins une huile hydrocarbonée et/ou une huile siliconée. Dans ces gels, l'élastomère émulsionnant est souvent sous forme de particules non-sphériques.

Des élastomères polyoxyalkylénés sont notamment décrits dans les brevets US5236986, US5412004, US5837793, US5811487.

Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-310", "KSG-320", "KSG-330", "KSG-340", "X-226146" par la société Shin Etsu, "DC9010", "DC9011" par la société Dow Corning.

Des élastomères polyglycérolés sont notamment décrits dans la demande WO-A-2004/024798.

Comme élastomère de silicone polyglycérolé, on peut utiliser ceux vendus sous les dénominations "KSG-710", "KSG-810", "KSG-820", "KSG-830", "KSG-840" par la société Shin Etsu.

Le silicone élastomère émulsionnant, à savoir l'organopolysiloxane réticulé élastomère, de préférence à chaîne polyhydroxylée, est présent dans la composition cosmétique de l'invention en une teneur comprise entre 0,05% et 3%, de préférence comprise entre 0,1% et 2%, les pourcentages étant des pourcentages en poids par rapport au poids total de la composition, étant entendu que les pourcentages de silicone élastomère émulsionnant sont exprimés en pourcentage de matière active.

Selon un mode de réalisation de l'invention, on utilise dans la composition cosmétique l'émulsionnant siliconé KF6038, seul ou associé avec le silicone élastomère émulsionnant KSG210.

### Huile non volatile

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

On entend par « huile non volatile » une huile qui présente une température d'ébullition généralement supérieure à 300°C sous 760 mm de Hg (101325 Pa) et qui ne présente pas ou peu de tension de vapeur.

### Huile siliconée non volatile

On entend par « huile siliconée » ou « huile de silicone », une huile comprenant au moins un atome de silicium, et notamment au moins un groupe Si-O.

Plus particulièrement selon un mode de réalisation de l'invention, l'huile siliconée non volatile est choisie dans le groupe comprenant les polydiméthylsiloxanes (PDMS) (diméthicones : nom INCI) renfermant au moins 8 atomes de silicium, les polyalkylméthylsiloxanes dont la chaîne alkyle renferme de 8 à 20 atomes de carbone, les alkyl diméthicones, les vinyl méthyl méthicones, les huiles identifiées par le nom INCI phényl triméthicones et leurs mélanges.

Selon un mode de réalisation de l'invention, l'huile siliconée non volatile présente une viscosité à 25°C supérieure ou égale à 5 cSt. De préférence l'huile siliconée non volatile présente une viscosité à 25°C inférieure ou égale à 10 cSt.

A titre d'exemple de diméthicone on pourra citer la « diméthicone 10cSt » commercialisée par :
- la société SHIN-ETSU sous la référence KF-96-A, et plus particulièrement KF-96-A-10CS,
- la société Wacker Chimie sous la référence Belsil® DM10,
- la société Dow Corning sous la référence Xiameter-PM-200 SIL FLUID 10CS,
- la société Evonik Industrie AG sous la référence Abil 10.

A titre d'exemple de diméthicone on pourra citer la « diméthicone 5cSt » commercialisée par Dow Corning sous la référence Xiameter PMX-200 SIL FLUID 5CS.

L'huile siliconée non volatile de l'invention peut être utilisée seule ou en mélange avec d'autres huiles non volatiles.

L'huile non volatile pouvant notamment être utilisée dans la composition de l'invention en mélange avec l'huile siliconée non volatile, est une huile ester non volatile.

On entend par « huile ester » non volatile, une huile comprenant au moins une fonction ester dans sa molécule. Avantageusement l'huile ester non volatile est un monoester.

L'huile ester non volatile peut être choisie parmi les monoesters de formule R₁COOR₂ dans laquelle R₁ représente une chaîne hydrocarbonée, linéaire ou ramifiée, comportant de 4 à 40 atomes de carbone, de préférence de 4 à 30 atomes de carbone, et de préférence de 7 à 20 atomes de carbone, et R2 représente une chaîne hydrocarbonée ramifiée contenant de 3 à 40 atomes de carbone, de préférence de 10 à 30 atomes de carbone, et de préférence de 16 à 26 atomes de carbone.

Comme exemple d'huile ester non volatile, on pourra citer de façon préférée le palmitate de 2-éthyl-hexyle, le palmitate d'isopropyle, le palmitate d'isooctyle, le palmitate d'isocétyle, le palmitate d'isodécyle, le palmitate d'isostéaryle, le palmitate de 2-octyl-décyle, le néopentanoate d'isodécyle, l'octanoate d'isocétyle, l'isononanoate d'isononyle, l'isononanoate d'isodécyle, l'isononanoate de tridécyle, le laurate d'hexyle, le laurate de 2-hexyl-décyle, le myristate d'isopropyle, le myristate d'isocétyle, le myristate d'isotridécyle, le myristate de 2-octyldodécyle, l'isostéarate d'isopropyle, le stéarate d'octyl-2 dodécyle, l'isostéarate d'isostéaryle, l'érucate d'octyl-2 dodécyle, le diisostéaryle malate et leurs mélanges.

En fonction des applications visées, d'autres huiles non volatiles pourront être ajoutées dans les compositions de l'invention.

Dans le contexte de la présente invention, le terme "huile" comprend également des graisses lipophiles qui sont capables de subir un changement liquide/solide réversible et ont une organisation cristalline anisotrope à l'état solide, mais qui sont différents des cires par le fait qu'elles contiennent, à une température de 23°C, une fraction liquide et une fraction solide. Un composé de ce type est notamment un mélange d'esters de stérols, tel que le mélange de cholestérol et d'ester de lanosterol disponibles auprès du fabricant CRODA sous la dénomination commerciale Super Sterol Ester®.

Les huiles utilisables dans la composition selon l'invention peuvent être choisies dans le groupe comprenant les huiles hydrocarbonées ; les (poly) esters synthétiques et (poly) éthers, en particulier les (poly) esters d'acides en C6-C20 et d'alcools en C6-C20, avantageusement ramifiés tels que l'isononanoate d'isononyle ; les huiles végétales ; les acides gras ramifiés et/ou insaturés ; les alcools gras ramifiés et/ou insaturés tels que l'octyldodécanol ; les huiles fluorosilicone ; les huiles fluorées ; et leurs mélanges.

Dans le cas où l'on souhaite obtenir une composition brillante, par exemple pour une ombre à paupière, un baume à lèvres brillant ou un rouge à lèvres brillant, il est préférable d'utiliser dans la présente invention au moins une huile brillante, c'est-à-dire une huile présentant un indice de réfraction supérieur à 1,45, de préférence supérieur à 1,47.

Des exemples d'huiles brillantes sont notamment les huiles hydrocarbonées telles que le squalane, le phytosqualane, le polybutène, le polyisobutène hydrogéné, le polydécène hydrogéné ; les huiles de silicone phénylées telles que celles identifiées :
- par le nom INCI "phényl triméthicone", notamment celle commercialisée par Rhodia sous la dénomination MIRASIL® PTM,
- par le nom INCI "phénylpropyldiméthylsiloxysilicate", notamment celle commercialisée par General Electric sous la dénomination Silshine® 151,
- par le nom INCI "triméthylpentaphényl trisiloxane", notamment celle commercialisée par Dow Corning sous la dénomination commerciale DC PH® 1555 HRI.

On peut également citer, à titre d'huiles brillantes :
- les silicones fluorées identifiées par le nom INCI "perfluorononyl diméthicone", notamment celle commercialisée par Phoenix Chemical, Inc sous la dénomination commerciale Pecosil® FS (FSU, FSL, etc.), et celle commercialisée par Biosil Technologies sous la dénomination commerciale Biosil Basics® (Fluorosil LF, 14, et similaire) ;
- les amino céramides identifiées par le nom INCI "phytosteryl / octyldodécyl lauroyl glutamate", notamment celle commercialisée par Ajinomoto sous la dénomination commerciale Eldew® PS203.

D'autres exemples d'huiles brillantes sont les huiles naturelles, en particulier l'huile de graine de ricin, l'huile de jojoba, l'huile de limnanthes ; les alcools gras ramifiés et/ou insaturés tels que l'octyldodécanol, les mono- et polyesters d'acides gras et/ou d'alcools gras dont la chaîne grasse comprend de 6 à 20 atomes de carbone, en particulier les mono- et les polyesters d'hydroxyacides et d'alcools gras, tels que le diisostéarylmalate, les esters d'acide benzoïque et d'alcools gras, tels que les benzoates d'alkyle en C12-C15, les polyesters de polyols et en particulier du (di)pentaérythritol, tels que le tétraisostéarate de pentaérythrityle, le pentaisononanoate de dipentaérythrityl et les esters C5-C9 de dipentaérythrityl, les esters de polyglycérol, tels que ceux identifiés par le nom INCI "bis-diglycéryl polyacyladipate-1" vendus par Sasol sous la dénomination commerciale Softisan® 645, ou le polyglycéryl-2 triisostearate vendu sous la dénomination commerciale Salacos 43N par Nisshin Oillio Group Ltd, les esters de triméthylolpropane, tel que le triéthylhexanoate de triméthylolpropane, vendu notamment par Kokyu Alcohol Kogyo sous la dénomination commerciale Kak® TTO, les esters de propylèneglycol, tel que le dibenzoate de propylèneglycol, vendu en particulier par Inolex sous la dénomination commerciale Lexfeel Shine®, les stéarates de stéaroyle isocétyle ; les polyesters d'huile de ricin hydrogénée, tels que les esters vendus par Kokyu Alcohol Kogyo sous les dénominations commerciales Risocast® DA-H et Risocast® DA-L.

Dans les cas où on souhaite obtenir une composition matte, comme par exemple un anti-cernes, un fond de teint ou un rouge à lèvre mat, les huiles utilisées dans la composition de l'invention sont des huiles non brillantes.

Des exemples d'huiles non brillantes appropriées dans les compositions cosmétiques de l'invention comprennent les (poly) esters et (poly) éthers synthétiques, en particulier les (poly) esters d'acides en C6-C20 et d'alcools en C6-C20, avantageusement ramifiés, tels que l'isononanoate d'isononyle et le néopentanoate d'isostéaryle ; les di(alkyl en C6-C20) carbonates tels que le dicaprylyl carbonate commercialisé par BASF sous la dénomination Cetiol CC ; les acides gras ramifiés et/ou insaturés ; les polyesters de polyols, en particulier de (di) pentaérythritol, tels que le tétraoctanoate de pentaérythritol, les huiles de silicone telles que les polydiméthylsiloxanes linéaires de viscosité supérieure ou égale à 5 cSt ; et leurs mélanges.

Selon un mode de réalisation, les compositions de l'invention comprennent un mélange des huiles ci-dessus citées.

Selon un mode de réalisation avantageux de l'invention, l'huile non volatile, utilisée en association avec l'huile siliconée non volatile, est choisie dans le groupe comprenant les huiles non volatiles brillantes et en particulier les mono- et polyesters d'acides gras et/ou d'alcools gras dont la chaîne grasse comprend de 6 à 20 atomes de carbone, en particulier les mono- et les polyesters d'hydroxyacides et d'alcools gras, tels que le diisostéaryl malate.

Selon un autre mode de réalisation avantageux de l'invention, l'huile non volatile, utilisée en association avec l'huile siliconée non volatile, est choisie dans le groupe comprenant les huiles non volatiles siliconées non brillantes et en particulier les polydiméthylsiloxanes linéaires de viscosité supérieure ou égale à 5 cSt.

A titre d'exemple de l'invention, l'huile siliconée non volatile utilisée dans la composition est associée à une huile ester non volatile, notamment le palmitate de 2-éthyl-hexyle et/ou le diisostéaryl malate.

L'huile non volatile est présente dans la composition cosmétique de l'invention en une teneur comprise entre 30% et 50%, les pourcentages étant des pourcentages en poids par rapport au poids total de la composition.

L'huile siliconée non volatile est présente dans la composition cosmétique de l'invention dans une quantité comprise entre 10% et 50%, les pourcentages étant des pourcentages en poids par rapport au poids total de la composition.

Selon un mode de réalisation particulièrement intéressant de l'invention, la composition comprend :
- un mélange émulsionnant comprenant au moins un émulsionnant siliconé présent dans une quantité comprise entre 0,1% et 5% et au moins un silicone élastomère émulsionnant ledit silicone élastomère étant présent dans une quantité comprise entre 0,05% et 3%,
- au moins une huile non volatile présente dans une quantité comprise entre 30% et 50% et comprenant au moins une huile non volatile siliconée dans une quantité comprise entre 10% et 50% et une seconde huile choisie parmi au moins une huile ester non volatile,
- 2% à 12%, de préférence 6% à 10%, d'un agent structurant de la phase huileuse,
- 30% à 50% d'eau,
- 0% à 2% et de préférence 0% d'une huile siliconée volatile et/ou d'une huile hydrocarbonée volatile, et
- optionnellement un filmogène dans une quantité inférieure ou égale à 10%;
les pourcentages définis ci-dessus étant des pourcentages en poids par rapport au poids total de la composition.

Selon un mode préféré de l'invention la composition comprend un filmogène.

Le filmogène est choisi notamment parmi les résines de silicone. Parmi celles-ci on peut citer notamment les résines de type trimethylsiloxysilicates comme par exemple celle commercialisée par la société Wacker Chemie AG sous la dénomination Belsil® TMS 803.

Le filmogène peut également être choisi parmi les polymères silicone acrylates. Parmi ceux-ci on peut citer par exemple les Acrylates/Polyirimethylsiloxymethacrylate Copolymer tel que celui commercialisé par la société Dow Corning sous la dénomination *Dow Corning*® FA 4002 ID Silicone Acrylate.

Le filmogène peut également être choisi parmi les polymères de type polybutenes comme par exemple celui commercialisé par la société Rossow sous la dénomination Parleam® HV.

Le filmogène peut également être choisi parmi les résines d'origine végétale comme par exemple la résine Shorea Robusta resin comme celle prédispersée dans l'octyldodecanol commercialisée par la société Kahlwax sous la dénomination Kahlresin® 6723.

Le filmogène peut également être choisi parmi un mélange des filmogènes précédemment cités.

Selon un mode de réalisation particulièrement avantageux, la composition de l'invention comprend, à titre de filmogène, un mélange de Belsil® TMS 803 et de Kahlresin® 6723.

Le filmogène, quand il n'est pas optionnel, est présent dans la composition de l'invention dans une quantité comprise entre 1% et 15%, de préférence entre 1% et 10%, les pourcentages étant des pourcentages en poids par rapport au poids total de la composition, étant entendu que lorsque les filmogènes sont pré-dispersés dans un solvant, le pourcentage correspondant au pourcentage de matière active.

Selon un mode de réalisation particulièrement avantageux, la composition de l'invention comprend, à titre de filmogène, un mélange de 7% de Belsil® TMS 803 et de 2% Kahlresin® 6723, les pourcentages étant des pourcentages en poids par rapport au poids total de la composition.

### Agent structurant de la phase huileuse

L'agent structurant de la phase huileuse comprend au moins une cire et/ou au moins un gélifiant huileux.

Le terme «cire» désigne une matière grasse à changement réversible liquide/solide, ayant une température de fusion supérieure à 30°C et généralement inférieure à 110°C, qui est liquide dans les conditions de préparation de la composition et qui présente une organisation cristalline anisotrope à l'état solide.

Le terme « gélifiant huileux », désigne une substance capable de solidifier ou de gélatiniser l'huile introduite dans la composition de l'invention.

Selon un mode de réalisation de l'invention, l'agent structurant est un mélange d'au moins une cire et d'au moins un gélifiant huileux.

Selon un autre mode de réalisation de l'invention, l'agent structurant est un mélange d'au moins deux cires.

La cire appropriée pour les compositions cosmétiques de l'invention comprend au moins une cire polaire et/ou au moins une cire apolaire.

Par cire polaire, on entend une cire comprenant au moins un hétéroatome tel que l'oxygène, l'azote, le silicium ou le phosphore.

En particulier, la cire polaire peut être choisie dans le groupe comprenant la cire d'abeille, la cire de carnauba, la cire de candelilla, la cire de coton, la cire de son de riz, la cire de baie, la cire d'insecte de chine, la cire de montan, la lanoline et ses dérivés alcools, acétylés, estérifiés, polyéthoxylés, la cire de kapok, la cire de canne à sucre, le laurate d'hexyle, la cire de jojoba, la cire shellac, l'éther de cholestérol polyéthoxylé, les cires d'abeille synthétiques commercialisées par Koster Keunen sous la dénomination commerciale Kester Wax K82H, et leurs mélanges.

On peut également citer les cires d'esters végétaux choisies dans le groupe comprenant le mélange d'esters de jojoba, de polyglycérine-3, de cire de fleur d'*Acacia decurrens* et de cire de graines de tournesol, ledit mélange étant commercialisé par Gattefosse sous la dénomination commerciale Acticire®, les esters de jojoba commercialisés par Floratech sous la dénomination commerciale Floraesters 60 ou Floraesters 70, les esters d'alkyle ou d'esters d'alkyle hydrogénés commercialisés par Sophim sous le nom commercial Photowax, comme par exemple les esters d'oléate de lauroyl hydrogéné commercialisés sous la dénomination Phytowax Olive 12L44.

Par cire apolaire, on entend une cire hydrocarbonée et/ou une cire siliconée.

On entend par « cire apolaire hydrocarbonée », une cire comprenant uniquement des atomes de carbone et d'hydrogène et ne comprenant pas d'hétéroatomes tels que l'oxygène, l'azote, le silicium ou le phosphore.

Des exemples de cires apolaires hydrocarbonées appropriées dans les compositions de l'invention comprennent la cire de polyéthylène, commercialisée par New Phase Technologies sous la dénomination Performalène 400 (P400) ou par Jeen International Corporation sous la dénomination Jeenate 3H, un mélange de polyéthylène linéaire de haut poids moléculaire et de copolymère éthylène/propylène, commercialisé par Safic-Alcan sous la dénomination Lipwax® PZ80-20, une cire synthétique commercialisée par Sasol sous la dénomination Sasol Wax C80, des cires synthétiques et des cires végétales, comme par exemple un mélange de cire synthétique et de cire de carnauba (Copernica cerifera) commercialisé par Strahl & Pitsch sous la dénomination Smart wax 202, un mélange de cire synthétique, de cire Candelilla et de cire de carnauba (Copernica cerifera) commercialisé par Strahl & Pitsch sous la dénomination Smartwax 7743S, les cires de Fischer Tropsch commercialisées par Cirebelle sous la dénomination Cirebelle 303, et leurs mélanges.

On entend par « cire apolaire siliconée », une cire comprenant un hétéroatome de silicium.

Des exemples de cires apolaires de silicium appropriées dans les compositions de l'invention comprennent la C20-24 alkyl diméthicone, commercialisée par Siltech sous la dénomination Silwax D2024, la C24-28 alkyle diméthicone, commercialisée par Evonik Industries AG sous la dénomination Abil Wax, et leurs mélanges.

Selon un mode de réalisation de l'invention, la cire est une cire apolaire ou un mélange de cires apolaires, ladite cire apolaire étant une cire apolaire hydrocarbonée ou un mélange de cires apolaires hydrocarbonées.

Plus particulièrement, selon encore un autre mode de réalisation de l'invention, la cire est choisie dans le groupe comprenant une cire de polyéthylène, seule ou en association avec une autre cire de polyéthylène, un mélange de cire de polyéthylène et de cire d'esters végétaux, un mélange de cire synthétique et de cire de carnauba, les esters de jojoba et leurs mélanges.

A titre d'exemples de cire, on pourra citer la cire de polyéthylène « Jeenate 3H », un mélange de cire de polyéthylène « Jeenate 3H » et de cire de polyéthylène « Performalene 400 », un mélange de cire de polyéthylène « Jeenate 3H » et de cire « Smart wax 202 », un mélange de cire de polyéthylène « Jeenate 3H » et d'esters de jojoba « Floraester 70 ».

Selon un mode de réalisation de l'invention, la cire est présente dans la composition cosmétique de l'invention en une teneur comprise entre 2% et 12%, de préférence entre 6% et 10%, les pourcentages étant des pourcentages en poids par rapport au poids total de la composition.

Le choix d'une part de la cire et d'autre part de sa quantité, es effectué de manière à obtenir une composition cosmétique présentant une dureté acceptable, une bonne stabilité, de bonnes propriétés sensorielles, tout en maintenant la température de chauffage du « bulk » la plus basse possible pendant le procédé de fabrication de ladite composition.

Le gélifiant huileux pouvant être compris dans la composition cosmétique de l'invention est choisi dans le groupe comprenant un polymère siliconé ; un copolymère cyclique de vinyl diméthicone/diméthicone ; une résine polyamide ou une résine poly (ester-amide) ; un copolymère de styrène et d'au moins une oléfine autre que le styrène ; un diamide d'acide N-acyl glutamique ; un ester de dextrine ; un sucroester et leurs mélanges.

A titre d'exemple d'un polymère siliconé approprié comme gélifiant huileux, on pourra citer un polymère siliconé obtenu par autopolymérisation en présence d'un catalyseur d'un organopolysiloxane fonctionnalisé par des groupes époxy et hydrosilylé, commercialisé par General Electric sous la dénomination Velvesil® 125.

A titre d'exemple d'un copolymère cyclique de vinyl diméthicone/diméthicone convenable comme gélifiant huileux, on pourra citer celui commercialisé par Jeen sous la dénomination commerciale Jeesilc® PS (y compris PS VH, PS VHLV, PS CM, PS CMLV et PS DM).

A titre d'exemples de résines de polyamide ou de résines de poly (ester-amide) appropriées comme gélifiants huileux, on pourra citer les polyamides à terminaison ester (ETPA), les poly (ester-amides) terminés par un ester (ETPEA), les polyamides à terminaison amide tertiaire (ATPA), les polyamides à terminaison polyalkylèneoxy (PAOPA) ou les polyéther polyamides (PEPA).

Des exemples de polyamides à terminaison ester (ETPA) sont ceux identifiés par le nom INCI "Ethylenediamine / Stearyl Dimer Dilinoleate Copolymer" et disponibles, par exemple, sous le nom commercial Uniclear® 100VG de la société Arizona Chemical.

Des exemples de poly (ester-amides) terminés par un ester (ETPEA) sont ceux identifiés par le nom INCI polyamide-8 qui sont des "Copolymères dimère dibenzoate d'éthylènediamine bis-stéaryl éthylènediamine / néopentylglycol / stéaryle" et disponibles, par exemple, sous la dénomination commerciale Oloecraft® LP-20- PA-MV de la société Croda.

Des exemples de polyamides à terminaison amide tertiaire (ATPA) sont ceux identifiés par le nom INCI "Ethylènediamine / Copolymère de diilinoate de dimère hydrogéné Bis-Di-C14-18 Alkyl Amide" et disponibles, par exemple, sous la dénomination commerciale Sylvaclear® A200V ou Sylvaclear® A2614V de la société Arizona Chemical ou ceux identifiés par le nom INCI "malate de diisostéaryle et bis-dioctadécylamide dimère acide dilinoleique / éthylènediamine" et disponibles, par exemple, sous la dénomination commerciale Haimalate PAM de la société Kokyu Alcohol Kogyo.

Des exemples de polyamides à terminaison polyalkylèneoxy (PAOPA) sont ceux identifiés par le nom INCI Polyamide-3 et disponibles, par exemple, sous la dénomination Sylvaclear® AF1900V, Sylvaclear® PE1800V et Sylvaclear® PA1200V de la société Arizona Chemical.

Des exemples de polyéther polyamides (PEPA) sont ceux identifiés par le nom INCI Polyamide-6 et disponibles, par exemple, sous la dénomination Sylvaclear® PE400V de la société Arizona Chemical.

A titre d'exemple de copolymères de styrène et d'au moins une oléfine autre que le styrène, appropriés comme gélifiant huileux, on pourra citer des copolymères, en particulier des copolymères séquencés, de styrène et d'au moins une oléfine autre que le styrène choisi parmi l'éthylène, le propylène, le butylène, le butadiène, l'isoprène et leurs mélanges. Ces copolymères sont vendus par Shell sous la dénomination commerciale Kraton® et peuvent notamment être un copolymère styrène-éthylène-propylène, styrène-éthylène-butylène, styrène-butadiène, styrène-isoprène, styrène-butadiène-styrène, styrène-isoprène-styrène ou un copolymère styrène-éthylène-butylène-styrène ou encore un copolymère éthylène/propylène/styrène ou butylène/éthylène/styrène ou tout mélange des copolymères susmentionnés, par exemple un mélange de copolymères éthylène/propylène/styrène et butylène/éthylène/styrène. Un exemple d'un mélange de copolymères utilisable selon l'invention est constitué des produits Dekagel® vendus par Jan Dekker et en particulier Dekagel® HV2004. D'autres exemples de copolymères de styrène utilisables dans la présente invention sont ceux vendus par Penreco sous les dénominations commerciales Versagel® M, ME, ML, MP, MC, MD et MN, notamment Versagel® ME, plus particulièrement Versagel® ME 2000.

A titre d'exemples de diamides d'acide N-acyl glutamique appropriés comme gélifiant huileux, on pourra notamment citer un diamide d'acide N-acyl glutamique ayant un groupe alkyle à chaîne droite tel que le dibutyl lauroyl glutamide et un diamide d'acide N-acyl glutamique ayant un groupe alkyle à chaîne ramifiée, tels que le dibutyl éthylhexanoyl glutamide. Le dibutyl lauroyl glutamide est disponible dans le commerce en tant que GP-1 et le dibutyl éthylhexanoyl glutamide est disponible dans le commerce sous le nom EB-21, et sont tous deux commercialisés par Ajinomoto.

A titre d'exemples d'esters de dextrine appropriés comme gélifiant huileux, on pourra citer les esters de dextrine et d'acides gras, tels que le palmitate de dextrine.

A titre d'exemples d'esters de saccharose appropriés comme gélifiant huileux, on pourra citer les esters de saccharose et d'acides gras, tels que le triacétate de tétrastéarate de saccharose disponible sous la dénomination commerciale Sisterna® A10E-C de la société Sisterna.

Selon un mode de réalisation avantageux de l'invention, on pourra citer plus particulièrement comme gélifiant huileux les esters de saccharose tels que le triacétate de tétrastéarate de saccharose, commercialisé par Sisterna sous la dénomination commerciale Sisterna® A10E-C.

Selon un mode de réalisation de l'invention, le gélifiant de la phase huileuse est présent dans la composition cosmétique de l'invention en une teneur comprise entre 2% et 12%, de préférence comprise entre 6 et 10%, les pourcentages étant des pourcentages en poids par rapport au poids total de la composition.

L'agent structurant de la phase huileuse est présent dans la composition cosmétique de l'invention en une teneur comprise entre 2% et 12%, de préférence comprise entre 6% et 10%, les pourcentages étant des pourcentages en poids par rapport au poids total de la composition.

La composition de l'invention est plus particulièrement caractérisée en ce qu'elle comprend :
- un mélange émulsionnant comprenant au moins un émulsionnant siliconé présent dans une quantité comprise entre 0,1% et 5% et au moins un silicone élastomère émulsionnant ledit silicone élastomère étant présent dans une quantité comprise entre 0,05% et 3%
- au moins une huile non volatile présente dans une quantité comprise entre 30% et 50% et comprenant au moins une huile non volatile siliconée et éventuellement une seconde huile choisie parmi au moins une huile ester non volatile,
- 2% à 12%, de préférence 6% à 10%, d'agent structurant de la phase huileuse tel que défini ci-dessus,
les pourcentages définis ci-dessus étant des pourcentages en poids par rapport au poids total de la composition.

### Eau

Selon un mode de réalisation avantageux, la composition cosmétique de l'invention comprend de l'eau, de préférence de l'eau déminéralisée, en une quantité comprise entre 30% et 50%, de préférence comprise entre 35% et 45%, et plus préférentiellement en une quantité d'environ 40%, les pourcentages étant des pourcentages en poids par rapport au poids total de la composition.

### Huile volatile

Selon un mode de réalisation avantageux de l'invention, la composition ne contient pas (0%) ou alors très peu (maximum 2% en poids par rapport au poids total de la composition) d'huile volatile.

Par "huile volatile", on entend une huile susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle à température ambiante et pression atmosphérique, en particulier ayant une pression de vapeur comprise entre 0,13 Pa et 40 000 Pa (0,001 à 300 mm de Hg), de préférence comprise entre 1,3 Pa et 13 000 Pa (0,01 à 100 mm de Hg), et plus préférentiellement encore comprise entre 1,3 Pa et 1 300 Pa (0,01 à 1000 mm de Hg).

Les huiles volatiles comprennent les huiles siliconées volatiles et/ou les huiles hydrocarbonées volatiles.

Les huiles siliconées volatiles éventuellement utilisées dans les compositions de l'invention sont linéaires ou cycliques, ont notamment de 2 à 7 atomes de silicium, éventuellement des groupes alkyle ou alkoxy ayant de 1 à 10 atomes de carbone, et présentent une viscosité, à température ambiante, inférieure à 5 cSt.

A titre d'exemples d'huile siliconée volatile on pourra plus particulièrement citer l'hexaméthylcyclotrisiloxane, l'octaméthylcyclotétrasiloxane, le décaméthylcyclopentasiloxane, le dodecaméthylcyclohexasiloxane, le cyclotétradiméthylsiloxane, le cyclopentadiméthylsiloxane, le cyclohexadiméthylsiloxane, l'hexaméthyldisiloxane, l'octaméthyltrisiloxane, l'hexylheptaméthyltrisiloxane, l'octylheptaméthyltrisiloxane, le décaméthyltétrasiloxane, le dodécaméthylpentasiloxane, l'heptaméthylhexyl trisiloxane, l'heptaméthyloctyl trisiloxane et leurs mélanges.

Concernant l'huile hydrocarbonée volatile on pourra plus particulièrement citer une huile hydrocarbonée à chaîne courte, les alcanes linéaires volatiles tels que par exemple décrits dans le document FR2933865

A titre d'exemples d'huile(s) hydrocarbonée(s) à chaîne courte on pourra notamment celle(s) choisie(s) dans le groupe comprenant l'isododécane, l'isodécane, l'isohexadécane et leurs mélanges.

A titre d'exemple d'alcanes linéaires volatiles, on pourra citer ceux ayant des chaînes hydrocarbonées en :
- C9-C17, C10-C14, tel qu'un mélange de undécane et tridécane, commercialisé par BASF Care Creations sous la dénomination Cetiol® Ultimate,
- C15-19, tel que ceux commercialisés par Seppic sous la dénomination Emogreen L15,
- C12-14, tel que ceux commercialisés par Biosynthis sous la dénomination Vegelight 1214LC.

Selon un mode de réalisation avantageux de l'invention, la composition telle que définie ci-dessus peut comprendre en outre un émulsionnant non siliconé, un humectant, une charge, une matière colorante , un agent conservateur et/ou du chlorure de sodium.

### Emulsionnant non siliconé

L'émulsionnant non siliconé est notamment choisi dans le groupe comprenant les polymères du type ester d'acide gras de glycol polyoxyalkyléné, tels que le Polysorbate 60 commercialisé par Croda sous la dénomination Tween 60, le Polysorbate 20 commercialisé par Croda sous la dénomination Tween 20, le Polysorbate 85 commercialisé par Seppic sous la dénomination Montanox 85, et leurs mélanges, et est de préférence l'émulsionnant identifié par le nom INCI Polysorbate 60.

La composition cosmétique de l'invention comprend de 0% à 5% d'émulsionnant non siliconé, de préférence de 0,1% à 1%, les pourcentages étant des pourcentages en poids par rapport au poids total de la composition.

### Humectant

On entend par humectant un composé comprenant au moins deux groupes hydrophiles, c'est-à-dire des groupes qui forment des liaisons hydrogène avec des molécules d'eau, comme par exemple des groupes hydroxyle, amine et/ou carboxyle.

Un humectant approprié dans la composition cosmétique de l'invention est notamment choisi dans le groupe comprenant le glycérol ; les glycols, tels que le propylèneglycol, le butylèneglycol et l'hexylèneglycol ; les alcools de sucre ou les polyols de sucre, tels que le sorbitol, le xylitol et le maltitol ; les polyols polymères tels que le polydextrose ; les alpha-hydroxyacides tels que l'acide lactique ; l'acide hyaluronique ; l'urée ; l'acide L-pyrrolidone carboxylique (PCA) ; et leurs mélanges.

Selon un mode de réalisation avantageux de l'invention, l'humectant est le glycérol et/ou les glycols.

La composition cosmétique de l'invention comprend de 0% à 10% d'humectant, de préférence de 1% à 6% d'humectant, les pourcentages étant des pourcentages en poids par rapport au poids total de la composition.

### Charge

On entend par charge les particules incolores ou blanches, minérales ou de synthèse, solides de toutes formes, qui se présentent sous une forme insoluble et dispersée dans le milieu de la composition, quelle que soit la température à laquelle la composition est fabriquée. Les charges peuvent être minérales ou organiques et de toute forme, plaquettaires, sphériques ou oblongues, quelle que soit la forme cristallographique (par exemple feuillet, cubique, hexagonale, orthorombique, etc). Les charges peuvent être constituées de plusieurs couches de nature chimique et/ou de forme physique différentes et peuvent être réalisées sous forme de lamelles revêtues de charges sphériques. Elles peuvent être modifiées en utilisant différents traitements de surface.

La charge pouvant être utilisée dans les compositions cosmétiques de l'invention est choisie dans le groupe comprenant le talc, le mica, la silice, le kaolin, le nitrure de bore, la lauroyl lysine, la séricite, les billes de cellulose, les billes de verre, l'amidon, l'amidon modifié par l'anhydride octénylsuccinique, les résines de silicone telles que le polyméthylsilsesquioxane disponible sous la dénomination Tospearl 2000B de Momentive Performance Materials, les poudres dérivées d'élastomères de silicone tels que l'élastomère de silicone revêtu avec une résine de silicone disponible sous la dénomination commerciale KSP100 ou KSP300 de ShinEtsu, et leurs mélanges.

La composition cosmétique de l'invention comprend de 0% à 25% de charge, de préférence de 2% à 10%, les pourcentages étant des pourcentages en poids par rapport au poids total de la composition.

Les charges se présentent avantageusement sous forme pulvérulente.

### Matière colorante

La composition peut également comprendre au moins une matière colorante choisie dans le groupe comprenant les colorants hydrosolubles, les colorants liposolubles, les particules ayant pour effet de colorer et/ou d'opacifier la composition, telles que les pigments, les nacres, les laques (colorants hydrosolubles adsorbés sur un support inorganique inerte), et leurs mélanges.

Ces matières colorantes peuvent éventuellement être traitées à la surface avec un agent hydrophobe tel que des silanes, des silicones, des savons d'acides gras, des phosphates fluoroalkyle en C9-C15, des copolymères acrylate/diméthicone, des copolymères mixtes phosphate fluoroalkyle en C9-C15/silicone, des lécithines, des cires de carnauba, du polyéthylène, du chitosane et éventuellement des acides aminés acylés, tels que la lauroyl lysine, le stéaroyl glutamate disodique et l'acyl glutamate d'aluminium.

Les pigments peuvent être inorganiques ou organiques et naturels ou synthétiques.

Des exemples de pigments inorganiques sont notamment des oxydes de fer, de titane ou de zinc, le bleu outremer, le bleu de prusse, le violet de manganèse, l'oxyde de chrome, ainsi que des pigments composites et des pigments goniochromatiques, nacrés, interférents, photochromiques ou thermochromiques, sans que cette liste soit limitante.

Parmi les pigments organiques utilisables dans l'invention, on peut citer le Red 7, Red 6, Yellow 5, Yellow 6, Blue 1, Red 22, Red 28, Red 30, Red 33, Red 36, le noir de carbone, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Lorsqu'elle comprend un ou plusieurs pigments, la composition cosmétique selon l'invention comprend en outre un agent dispersant. A titre d'exemple d'agent dispersant on pourra citer le diisostéaryle malate, qui est également une huile ester.

Les nacres (ou agents nacrants) peuvent être choisies parmi celles classiquement présentes dans les produits de maquillage, tels que les produits à base de mica/dioxyde de titane. Elles peuvent encore être des produits à base de mica/silice/dioxyde de titane, à base de fluorophlogopite synthétique/dioxyde de titane (Sunshine® de Maprecos), à base de borosilicate de calcium sodique/dioxyde de titane (Reflecks® d'Engelhard) ou à base de borosilicate de calcium et d'aluminium/silice/dioxyde de titane (Ronastar® de Merck).

La composition cosmétique de l'invention comprend de 0% à 10% de matière colorante, de préférence de 2% à 6%, les pourcentages étant des pourcentages en poids par rapport au poids total de la composition.

Tout comme les charges, les matières colorantes se présentent avantageusement sous forme pulvérulente.

### Agent conservateur

La composition cosmétique peut également comprendre un agent conservateur, en particulier choisi dans le groupe comprenant le phénoxyéthanol, le sorbate de potassium, le déhydroacétate de sodium, la chlorphénésine, les parabènes tels que le méthylparabène ou le propylparabène, et leurs mélanges.

La composition cosmétique de l'invention comprend de 0% à 2% d'agent conservateur, de préférence de 0,1% à 1%, les pourcentages étant des pourcentages en poids par rapport au poids total de la composition.

### Chlorure de sodium

La composition cosmétique peut également comprendre du chlorure de sodium afin de stabiliser encore cette dernière. Le chlorure de sodium sera présent en une quantité comprise entre 0% et 1,5%, de préférence comprise entre 0,3% et 0,7% en poids par rapport au poids total de la composition.

Selon un mode de réalisation avantageux de l'invention, la composition telle que définie ci-dessus est plus particulièrement caractérisée en ce qu'elle comprend :
- 0% à 5% d'émulsionnant non siliconé tel que défini ci-dessus, de préférence 0,1% à 1%,
- 0% à 10% d'humectant tel que défini ci-dessus, de préférence 1% à 6%,
- 0% à 25% de charge tel que définie ci-dessus, de préférence 2% à 10%,
- 0% à 10% de matière colorante tel que définie ci-dessus, de préférence 2% à 6%,
- 0% à 2% d'agent conservateur tel que défini ci-dessus, de préférence 0,1% à 1%,
- 0% à 1,5% de chlorure de sodium, de préférence de 0,3 à 0,7%,
les pourcentages définis ci-dessus étant des pourcentages en poids par rapport au poids total de la composition.

### Actif cosmétique

La composition selon l'invention peut par ailleurs comprendre un actif cosmétique, en particulier choisi dans le groupe comprenant les agents hydratants tels que l'acide hyaluronique, l'acide L-pyrrolidone carboxylique (PCA); les agents cicatrisants tels que l'allantoine ; les agents anti-âges tels que les anti-oxydants, comme par exemple l'acide ascorbique et/ou ses esters alkyle ou phosphoryle, le tocophérol et/ou ses esters ; et leurs mélanges.

### Agent séquestrant

La composition de l'invention peut également comprendre un agent séquestrant, tel que les sels de l'acide éthylène-diamine-tétraacétique acide édétique (EDTA).

### Filtre UV

La composition de l'invention peut en outre comprendre un filtre UV choisi dans le groupe comprenant les filtres organiques, les filtres inorganiques et leurs mélanges.

On peut notamment citer, à titre de filtres organiques, les dérivés de dibenzoylméthane (comprenant le butylméthoxydibenzoylméthane), les dérivés d'acide cinnamique (comprenant le méthoxycinnamate d'éthylhexyle), les salicylates, les acides para-aminobenzoïques, les β,β-diphénylacrylates, les benzophénones, les dérivés de benzylidènecamphore, les phénylbenzimidazoles, les triazines, les phénylbenzotriazoles et/ou les dérivés anthraniliques.

On peut notamment citer, à titre de filtres inorganiques, les filtres à base d'oxydes inorganiques sous forme de pigments ou de nanopigments qui peuvent être ou non enrobés, et en particulier à base de dioxyde de titane ou d'oxyde de zinc.

### Agent édulcorant

Lorsque la composition de l'invention se présente sous forme d'un produit pour les lèvres elle peut en outre comprendre un agent édulcorant, notamment choisi dans le groupe comprenant le sorbitol, le sucrose, le xylitol, l'acésulfame-K, le saccharinate de sodium et leurs mélanges.

Lorsque la composition cosmétique de l'invention se présente sous la forme d'un anti-cernes, elle peut comprendre en outre un agent astringent (qui diminue l'apparence des pores) et/ou un agent de blanchiment.

Des exemples d'additifs pouvant être ajoutés dans les compositions cosmétiques de l'invention sont notamment décrits dans le dictionnaire CTFA (International Cosmetic Ingrédient Dictionary) et dans le Handbook publié par « The Cosmetic, Toiletry and Fragrance Association, 9th Edition, 2002 ».

Bien entendu l'homme du métier veillera à choisir ce ou ces éventuels ingrédients cosmétiques usuels complémentaires, et/ou leur quantité, de manière telle que les propriétés avantageuses de la composition selon l'invention ne soient pas, ou substantiellement pas, altérées par l'adjonction envisagée.

La composition de l'invention se présente sous la forme d'une émulsion eau-dans-huile solide stable, avec un aspect homogène.

On entend par stable, une émulsion qui ne relargue pas d'eau ou d'huile et qui reste sous sa forme initiale, même exposée aux étuves à une température comprise entre 5°C et 40°C pendant 3 mois.

On entend par aspect homogène, une émulsion dont la surface est lisse et uniforme à l'œil nu.

La composition cosmétique de l'invention telle que définie ci-dessus peut encore être caractérisée en ce qu'elle présente une dureté comprise entre 70 g et 150 g (grammes), de préférence comprise entre 80 g et 120 g, et plus préférentiellement encore comprise entre 80 g et 100 g.

La dureté de la composition, qui est exprimée en grammes (g), est déterminée par la mesure de la force de compression mesurée à 20°C à l'aide d'un texturomètre vendu sous la dénomination « TA-XT Plus Microstable System » par la société Swantech. Le texturomètre est équipé d'un cylindre en inox d'un diamètre de 2 mm se déplaçant à la vitesse de mesure de 1mm/s et pénétrant dans la composition à une profondeur de 3 mm.

La valeur de la dureté est la force de compression mesurée, divisée par la surface du cylindre du texturomètre en contact avec la composition. Les échantillons sont coulés à chaud et à ras bord dans une boite de pétri ronde de taille 60 mm de rayon et 15 mm de hauteur. Les échantillons ainsi préparés sont conservés pendant 24 h à 48 h à 20°C avant d'effectuer la mesure.

La composition selon l'invention peut être avantageusement utilisée pour le soin non thérapeutique et/ou le maquillage de la peau ou des lèvres.

Selon ce mode de réalisation, l'invention concerne une composition cosmétique sous la forme d'une émulsion eau-dans-huile solide telle que définie ci-dessus, caractérisée en ce qu'elle se présente sous la forme d'un stick, de préférence d'un stick de maquillage pour la peau ou les lèvres et/ou d'un stick de soin non thérapeutique pour la peau ou les lèvres.

L'invention a également pour objet un procédé cosmétique de maquillage et/ou de soin non thérapeutique de la peau ou des lèvres, caractérisé par le fait qu'il consiste à appliquer sur la peau ou les lèvres une composition telle que définie ci-dessus.

La composition selon l'invention peut être préparée selon le mode opératoire suivant.

On prépare d'une part le mélange des constituants de la phase huileuse en mélangeant et en chauffant, à une température comprise entre 70°C et 120°C, le système émulsionnant siliconé (à savoir l'émulsionnant siliconé et éventuellement le silicone élastomère émulsionnant), l'huile siliconée non volatile et l'agent structurant de la phase huileuse.

Le mélange est agité et maintenu à une température comprise entre 70°C et 95°C.

Des ingrédients optionnels tels que d'autres huiles non volatiles, des matières colorantes etc. peuvent être ajoutés dans la phase huileuse.

On prépare d'autre part la phase aqueuse comprenant majoritairement de l'eau, et éventuellement un émulsionnant non siliconé, et des conservateurs.

On chauffe ensuite la phase aqueuse à une température comprise entre 70°C et 95°C, puis on ajoute la phase aqueuse dans la phase huileuse, on agite à l'aide d'une turbine jusqu'à l'obtention de l'émulsion eau-dans-huile.

L'émulsion est ensuite coulée, à une température comprise entre 55°C et 95°C dans un contenant approprié afin de lui donner la forme d'un stick, puis est refroidie à température ambiante jusqu'à l'obtention de l'émulsion solide.

Les exemples suivants sont donnés à titre illustratif et non limitatif de la présente invention.

### Exemples

Six émulsions eau-dans-huile solide pour les lèvres ayant les compositions respectives données dans les tableaux 1 à 6 ci-dessous (compositions 1 à 6) ont été préparées.

Les pourcentages indiqués sont des pourcentages en poids.

### 1) Composition 1

**Tableau 1 :**

| **Nom INCI** | **Nom commercial** | **Qté en %** (Total = 100%) | **Fonction** |
|---|---|---|---|
| Diméthicone | KF-96A-10CS | 35 | Huile siliconée non volatile |
| Synthetic wax & cera carnauba (copernicia cerifera (carnauba) wax) | Smart wax 202 | 5 | Cire |
| Jojoba esters & polyglycerin-3 & acacia decurrens flower wax & helianthus annuus cera seed (helianthus annuus (sunflower) seed wax) | Acticire | 4,5 | Cire |
| Lauryl PEG-9 polydimethylsiloxyethyl dimethicone | KF-6038 | 2,5 | Emulsionnant siliconé |
| Dimethicone & dimethicone/PEG-10/15 crosspolymer & dipropylene glycol & tocopherol* | KSG-210 | 2 | Silicone élastomère émulsionnant |
| Pigment = mélange d'oxyde de fer et de dioxyde de titane | - | 2 | Pigment broyé et dispersé dans l'huile ester |
| Diisostearyl malate | | 4 | |
| | | 3,8 | Nacres |
| Aqua (water) | Eau déminéralisée | 40 | Eau |
| Polysorbate 60 | Tween 60-LQ | 0,5 | Emulsionnant non siliconé |
| Phenoxyethanol | Sepicide LD | 0,7 | Conservateur |

| | | | |
|---|---|---|---|
| *: Le silicone élastomère émulsionnant KSG-210 ayant pour nom INCI " Dimethicone & Dimethicone/PEG-10/15 Crosspolymer & Dipropylene glycol & tocopherol" se décompose de la manière suivante: 74,5% de Dimethicone; 25% de Dimethicone/PEG-10/15 Crosspolymer; 0,5% de Dipropylene glycol; 0,02% de tocopherol. | | | |

Donc 2% de KSG210 se décompose de la façon suivante: 1,5% de Dimethicone; 0,5% de Dimethicone/PEG-10/15 Crosspolymer; 0,01% de Dipropylene glycol; 0,0004% de tocopherol.

La quantité de "matière active" de silicone élastomère émulsionnant dans 2% de KSG-210 est donc de 0,5%.

### Remarque :

L'ajout d'agent dispersant diisostearyl malate (qui est aussi une huile ester) permet d'améliorer la dispersion des pigments dans la composition.

Le mode opératoire mis en œuvre pour la préparation de la composition 1 (tableau 1) ci-dessus est le suivant.
1) La phase grasse est préparée au bain marie à 95°C. Plus particulièrement, on réalise un pré-mélange du silicone élastomère émulsionnant KSG-210 dans l'émulsionnant siliconé KF-6038. On ajoute le broyage des pigments (dispersé dans le diisosterayl malate) et l'huile siliconée non volatile (KF-96A-10CS). Le mélange est agité manuellement.
2) Les cires sont ajoutées au mélange obtenu en 1). Le mélange est chauffé à une température comprise entre 90°C et 100°C et est maintenu à cette température pendant 10 minutes.
3) Le mélange obtenu en 2) est mis sous agitation rayneri à 300 rpm, puis les nacres sont ajoutées.
4) En parallèle la phase aqueuse est préparée. L'émulsionnant non siliconé (Tween 60-LQ) est solubilisé dans l'eau déminéralisée et on ajoute le conservateur. L'ensemble est chauffé au bain-marie à une température de 90°C.
5) La phase aqueuse obtenue en 4) est versée, lentement et de manière continue, dans la phase grasse obtenue en 3) sous agitation forte.
6) Le mélange obtenu en 5) est laissé émulsionné pendant 5 minutes en maintenant la température à 90°C
7) L'émulsion eau-dans-huile ainsi obtenue est coulée à chaud à une température comprise entre 70°C et 90°C dans un dispositif approprié pour préparer un stick à lèvres.
8) Le produit coulé est laissé refroidir avant le démoulage.

### 2) Composition 2

**Tableau 2 :**

| **Nom INCI** | **Nom commercial** | **Qté en % (Total = 100%)** | **Fonction** |
|---|---|---|---|
| Dimethicone & Dimethicone/PEG-10/15 Crosspolymer & Dipropylene glycol & tocopherol | KSG-210 | 2 | Silicone élastomère émulsionnant |
| Lauryl PEG-9 polydimethylsiloxyethyl dimethicone | KF-6038 | 2,5 | Emulsionnant siliconé |
| Pigment = mélange d'oxyde de fer et dioxyde de titane | - | 2 | Pigment broyé et dispersé dans l'huile ester |
| Diisostearyl malate | | 4 | |
| Diméthicone | KF-96A-10CS | 28,275 | Huile siliconée non volatile |
| Ethylhexyl palmitate | Palmitate ethyle 2 hexyle | 9,425 | Huile ester non volatile |
| Polyethylene | Jeenate 3H | 6 | Cire |
| Polyethylene | Performalene 400 | 1,5 | Cire |
| | | 3,8 | Nacres |
| Aqua (water) | Eau déminéralisée | 40 | Eau |
| Polysorbate 60 | Tween 60-LQ | 0,5 | Emulsionnant non siliconé |

### Remarque :

L'huile ester non volatile utilisée dans la composition de l'invention, à savoir le palmitate de 2-éthyl-hexyle, permet d'améliorer la compatibilité de la cire hydrocarbonée avec les composés siliconés de la formule.

Le mode opératoire mis en œuvre pour la préparation de la composition 2 (tableau 2) ci-dessus est le même que celui de l'exemple 1. Le palmitate de 2-ethyl-hexyle est ajouté dans la phase grasse du point 1).

La composition 2 est par la suite coulée dans une boite de Pétri et placée pendant 24h à 48h à 20°C avant d'effectuer la mesure de la dureté dans les conditions ci-dessus décrites.

La mesure de la dureté de la composition 2 (tableau 2) est de 88 +/- 3 g.

### 3) Composition 3

**Tableau 3:**

| **Nom INCI** | **Nom commercial** | **Qté en %** (Total = 100%) | **Fonction** |
|---|---|---|---|
| Dimethicone & Dimethicone/PEG-10/15 Crosspolymer & Dipropylene glycol & tocopherol | KSG-210 | 2 | Silicone élastomère émulsionnant |
| Lauryl PEG-9 polydimethylsiloxyethyl dimethicone | KF-6038 | 2,5 | Emulsionnant siliconé |
| Pigment = mélange d'oxyde de fer et de dioxyde de titane | - | 2 | Pigment broyé dispersé dans l'huile ester |
| Diisostearyl malate | | 4 | |
| Diméthicone | KF-96A-10CS | 27,775 | Huile siliconée non volatile |
| Ethylhexyl palmitate | Palmitate ethyle 2 hexyle | 9,425 | Huile ester non volatile |
| Polyethylene | Jeenate 3H | 6 | Cire |
| Synthetic wax & cera carnauba (copernicia cerifera (carnauba) wax) | Smart Wax 202 | 2 | Cire |
| | | 3,8 | Nacres |
| Aqua (water) | Eau déminéralisée | 40 | Eau |
| Polysorbate 60 | Tween 60-LQ | 0,5 | Emulsionnant non siliconé |

Le mode opératoire mis en œuvre pour la préparation de la composition 3 (tableau 3) ci-dessus est le même que celui décrit à l'exemple 1.

La composition 3 est par la suite coulée dans une boite de Pétri et placée pendant 24 h à 48 h à 20°C avant d'effectuer la mesure de la dureté dans les conditions ci-dessus décrites.

La dureté de la composition 3 (tableau 3) est de 80 +/- 2 g

### 4) Composition 4

**Tableau 4:**

| **Nom INCI** | **Nom commercial** | **Qté en %** (Total = 100%) | **Fonction** |
|---|---|---|---|
| Dimethicone & Dimethicone/PEG-10/15 Crosspolymer & Dipropylene glycol & tocopherol | KSG-210 | 2 | Silicone élastomère émulsionnant |
| Lauryl PEG-9 polydimethylsiloxyethyl dimethicone | KF-6038 | 2,5 | Emulsionnant siliconé |
| Pigment = mélange d'oxyde de fer et de dioxyde de titane | - | 2 | Pigment broyé dispersé dans l'huile ester |
| Diisostearyl malate | | 4 | |
| Diméthicone | KF-96A-10CS | 26,775 | Huile siliconée non volatile |
| Ethylhexyl palmitate | Palmitate ethyle 2 hexyle | 9,425 | Huile ester non volatile |
| Polyethylene | Jeenate 3H | 9 | Cire |
| | | 3,8 | Nacres |
| Aqua (water) | Eau déminéralisée | 40 | Eau |
| Polysorbate 60 | Tween 60-LQ | 0,5 | Emulsionnant non siliconé |

Le mode opératoire mis en œuvre pour la préparation de la composition 4 (tableau 4) ci-dessus est le même que celui décrit dans l'exemple 1.

La composition 4 est par la suite coulée dans une boite de Pétri et placée pendant 24 h à 48 h à 20°C avant d'effectuer la mesure de la dureté dans les conditions ci-dessus décrites.

La dureté de la composition 4 (tableau 4) est de 112 +/- 4 g

### 5) Composition 5

**Tableau 5 :**

| **Nom INCI** | **Nom commercial** | **Qté en %** (Total = 100%) | **Fonction** |
|---|---|---|---|
| Dimethicone & Dimethicone/PEG-10/15 Crosspolymer & Dipropylene glycol & tocopherol | KSG-210 | 2 | Silicone élastomère émulsionnant |
| Lauryl PEG-9 polydimethylsiloxyethyl dimethicone | KF-6038 | 2,5 | Emulsionnant siliconé |
| Pigment = mélange d'oxyde de fer et de dioxyde de titane | - | 2 | Pigment broyé et dispersé dans l'huile ester |
| Diisostearyl malate | | 4 | |
| Diméthicone | KF-96A-10CS | 27,775 | Huile siliconée non volatile |
| Ethylhexyl palmitate | Palmitate ethyle 2 hexyle | 9,425 | Huile ester non volatile |
| Polyethylene | Jeenate 3H | 6 | Cire |
| Jojoba esters | Floraesters 70 | 2 | Cire |
| | | 3,8 | Nacres |
| Aqua (water) | Eau déminéralisée | 40 | Eau |
| Polysorbate 60 | Tween 60-LQ | 0,5 | Emulsionnant non siliconé |

Le mode opératoire mis en œuvre pour la préparation de la composition 5 (tableau 5) ci-dessus est le même que celui décrit dans l'exemple 1 ci-dessus, à la différence que la température d'émulsification de l'étape 6 et la température de coulage de l'étape 8 sont abaissées à une température comprise entre 70°C et 80°C grâce au mélange de cire de polyéthylène « Jeenate 3H » et d'esters de jojoba « Floraester 70 ».

La composition 5 est par la suite coulée dans une tabatière et placée pendant 24h à 48h à 20°C avant d'effectuer la mesure de la dureté dans les conditions ci-dessus décrites.

La dureté de la composition 5 (tableau 5) est de 86 +/- 5 g.

### 6) Composition 6

**Tableau 6 :**

| **Nom INCI** | **Nom commercial** | **Qté en %** (Total = 100%) | **Fonction** |
|---|---|---|---|
| Dimethicone & Dimethicone/PEG-10/15 Crosspolymer & Dipropylene glycol & tocopherol | KSG-210 | 2 | Silicone élastomère émulsionnant |
| Lauryl PEG-9 polydimethylsiloxyethyl dimethicone | KF-6038 | 2,5 | Emulsionnant siliconé |
| Pigment = mélange d'oxyde de fer et de dioxyde de titane | - | 5 | Pigment broyé et dispersé dans l'huile ester |
| Diisostearyl malate | | 7,3 | |
| Diméthicone | XIAMETER PMX-200 silicone fluide-5CS | 13,2 | Huile siliconée non volatile |
| Diisostearyl malate | Salacos 222 | 3 | Huile ester non volatile |
| Ethylhexyl palmitate | Palmitate ethyle 2 hexyle | 8 | Huile ester non volatile |
| Polyethylene | Jeenate 3H | 6 | Cire |
| Jojoba esters | Floraesters 70 | 2 | Cire |
| Jojoba esters & polyglycerin-3 & Acacia decurrens flower wax & Helianthus annuus cera seed | Acticire MB | 0,1 | Cire |
| Sodium chloride | | 0,5 | additif |
| Trimethylsiloxysilicate | Belsil TMS 803 | 7 | filmogène |
| Shorea Robusta Resin octyldodecanol | Kahlresin® 6723 | 1 | filmogène |
| | | 1 | |
| Aqua (water) | Eau déminéralisée | 39,2 | Eau |
| phenoxyethanol | | 0,8 | conservateur |
| Anti-oxydant et parfum | | 0.7 | Anti-oxydant et parfum |
| actif | | 0,2 | actif |
| Polysorbate 60 | Tween 60-LQ | 0,5 | Emulsionnant non siliconé |

Le mode opératoire mis en œuvre pour la préparation de la composition 6 (tableau 6) ci-dessus est le suivant :
1) on pré-disperse le filmogène « Belsil TMS 803 » dans l'huile siliconée non volatile « Xiameter Pmx-200 Sil Fluid 5CS » jusqu'à obtenir un mélange transparent et homogène.
2) La phase grasse est préparée au bain marie à une température comprise entre 70°C et 80°C. Plus particulièrement on mélange l'huile ester non volatile « palmitate ethyle 2 hexyle », les cires « Jeenathe 3H », « Floraesters 70 », « Acticire MB » et le filmogène « Kahlresin® 6723» sous faible agitation.
3) On réalise un pré-mélange du silicone élastomère émulsionnant « KSG-210 » avec l'émulsionnant siliconé « KF-6038 », puis on ajoute ce pré-mélange d'émulsionnants au mélange de l'étape 2 en maintenant la température entre 70 et 80°C et en agitant environ 10mn sous une agitation forte d'environ 3000 rpm (rotation par minute) dans un appareil IKA.
4) On ajoute ensuite le mélange de l'étape 1 au mélange de l'étape 3 en maintenant la température entre 70 et 80°C et en agitant environ 10mn sous une agitation forte d'environ 3000 rpm.
5) On diminue l'agitation et on maintient la température entre 70°C et 80°C pour introduire les pigments qui ont été pré-dispersés dans du diisostaryl malate.
6) En parallèle la phase aqueuse est préparée. L'émulsionnant non siliconé (Tween 60-LQ) est solubilisé dans l'eau déminéralisée et on ajoute le conservateur et le chlorure de sodium. L'ensemble est chauffé au bain marie à une température comprise entre 70°C et 80°C.
7) La phase aqueuse obtenue en 6) est versée, lentement et de manière continue, dans la phase grasse obtenue en 5) sous agitation forte.
8) Le mélange obtenu en 7) est laissé émulsionné pendant 5 minutes en maintenant la température à une température comprise entre 70°C et 80°C.
9) On ajoute les ingrédients sensibles à la chaleur tels que les anti-oxidants, actifs et parfums.
10) L'émulsion eau-dans-huile ainsi obtenue à l'étape 9 est coulée à chaud à une température comprise entre 70°C et 80°C dans un dispositif approprié pour préparer un stick à lèvres.
11) Le produit coulé est laissé refroidir avant le démoulage.

La composition 6 est par la suite coulée dans une boîte étanche et placée pendant 24h à 48h à 20°C avant d'effectuer la mesure de la dureté dans les conditions ci-dessus décrites.

La dureté de la composition 6 (tableau 6) est de 100 +/- 4g.

### Conclusions

Le choix particulier des émulsionnants siliconés, des huiles siliconées non volatiles (et éventuellement d'autres huiles non volatiles, des silicones élastomères émulsionnants), des cires utilisées dans les compositions de l'invention permet d'obtenir une émulsion eau-dans-huile solide particulièrement stable, avec un aspect homogène et une bonne dureté (de 70 g à 150 g).

Les sticks à lèvres des tableaux 1 à 6 ci-dessus (compositions 1 à 6) ont été appliqués sur les lèvres d'un panel de 9 personnes.

Chacun de ces sticks à lèvres présentent d'excellentes propriétés de sensorialité, à savoir une sensation de confort, de fraîcheur et de fondant au moment de l'application sur la peau ou les lèvres. Ils présentent également une facilité d'étalement au moment de l'application sur la peau ou les lèvres et un résultat homogène une fois la composition appliquée. Par ailleurs la conservation des sticks à lèvres pendant une durée de 3 mois à une température de 45°C a permis d'observer que le stick présente une bonne stabilité dans le temps, à savoir qu'il ne rétrécit pas par rapport à sa forme initiale, qu'il ne se rompt pas à sa base, qu'il ne vieillit pas par dessèchement ou dessiccation et qu'il ne présente pas de phénomène d'exsudation (à savoir le relargage d'huile ou d'eau en surface).

En outre, au bout de 3 mois à 45°C, ces sticks à lèvres conservent d'excellentes propriétés de sensorialité, à savoir une sensation de confort, de fraîcheur et de fondant au moment de l'application sur la peau ou les lèvres. Ils conservent également une facilité d'étalement au moment de l'application sur la peau ou les lèvres et un résultat homogène une fois la composition appliquée.

## Revendications

1. Composition cosmétique sous la forme d'une émulsion eau-dans-huile solide **caractérisée en ce qu'**elle comprend :
- un mélange émulsionnant comprenant au moins un émulsionnant siliconé présent dans une quantité comprise entre 0,1% et 5% et au moins un silicone élastomère émulsionnant ledit silicone élastomère étant présent dans une quantité comprise entre 0,05% et 3%,
- au moins une huile non volatile présente dans une quantité comprise entre 30% et 50% et comprenant au moins une huile non volatile siliconée et éventuellement une seconde huile choisie parmi au moins une huile ester non volatile,
- un agent structurant de la phase huileuse comprenant une cire et/ou un gélifiant huileux,
- 30% à 50% d'eau,
- 0% à 2%, et plus préférentiellement encore 0% d'une huile siliconée volatile et/ou d'une huile hydrocarbonée volatile, et
- optionnellement un filmogène ;
les pourcentages définis ci-dessus étant des pourcentages en poids par rapport au poids total de la composition.

2. Composition selon la revendication 1, **caractérisée en ce que** l'émulsionnant siliconé est un polymère de siloxane comprenant :
- une chaîne latérale grasse,
- une chaîne latérale oxyéthylénée ou oxypropylénée et/ou une chaîne latérale polyéthoxylée, et éventuellement
- une chaîne latérale siliconée.

3. Composition selon la revendication 1 ou la revendication 2, **caractérisée en ce que** l'émulsionnant siliconé est choisi dans le groupe comprenant :
- le composé de formule (I) : 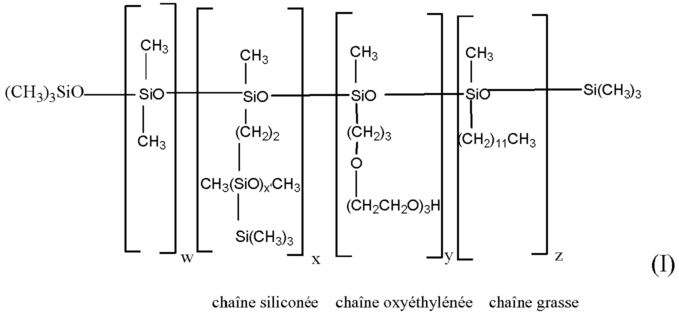 dans laquelle w est un entier compris entre 1 et 1000, x' est un entier compris entre 1 et 50, x, y et z représentent indépendamment les uns des autres un entier compris entre 1 et 100,
- le composé de formule (II) : 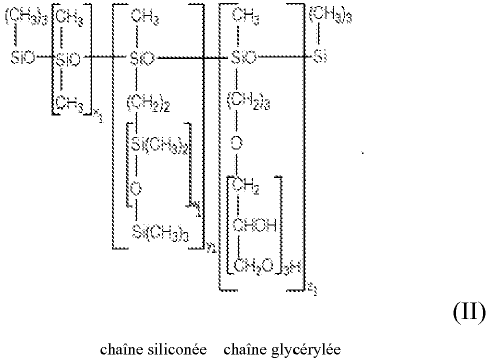 dans laquelle x₁ est un entier compris entre 1 et 1000, w₁ est un entier compris entre 1 et 50, y₁ et z₁ représentent indépendamment l'un de l'autre un entier compris entre 1 et 100,
- le composé de formule (III) : 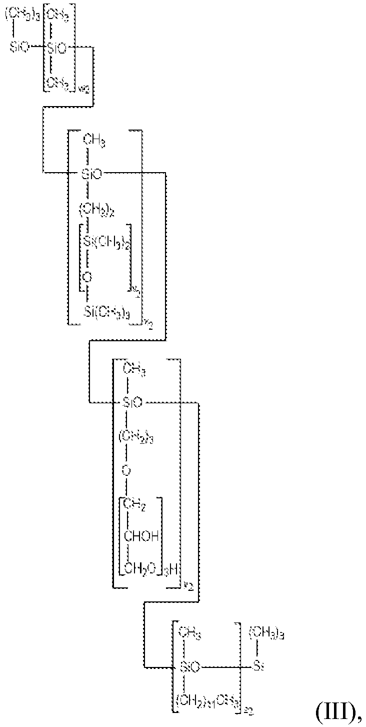 dans laquelle w₂ est un entier compris entre 1 et 1000, v₂ est un entier compris entre 1 et 50, x₂, y₂ et z₂ représentent indépendamment les uns des autres un entier compris entre 1 et 100,
et leurs mélanges.

4. Composition selon l'une quelconque des revendications 1 a 3, **caractérisée en ce qu'**elle comprend un silicone élastomère émulsionnant choisi parmi les organopolysiloxanes réticulés élastomères, de préférence à chaîne polyhydroxylée.

5. Composition selon l'une quelconque des revendications 1 à 4, **caractérisée en ce que** l'huile siliconée non volatile est choisie dans le groupe comprenant les polydiméthylsiloxanes (PDMS) renfermant au moins 8 atomes de silicium, les polyalkylméthylsiloxanes dont la chaîne alkyle renferme de 8 à 20 atomes de carbone, les alkyl diméthicones, les vinyl méthyl méthicones, les huiles identifiées par le nom INCI phényl triméthicones et leurs mélanges.

6. Composition selon l'une quelconque des revendications 1 à 5, **caractérisée en ce que** le filmogène est choisi parmi les résines de silicone et en particulier les trimethylsiloxysilicates, les silicones acrylates et en particulier les Acrylates/Polytrimethylsiloxymethacrylate Copolymer, les polybutenes ou les résines d'origine végétale telle que la Shorea Robusta Resin ou leur mélanges.

7. Composition selon l'une quelconque des revendications 1 à 6 **caractérisée en ce que** la quantité d'huile siliconée non volatile est comprise entre 10% et 50%, et la quantité de filmogène quand il est présent est comprise entre 1% et 15%, de préférence comprise entre 1% et 10%, les pourcentages définis ci-dessus étant des pourcentages en poids par rapport au poids total de la composition.

8. Composition cosmétique l'une quelconque des revendications 1 à 7 **caractérisée en ce que** l'huile siliconée non volatile présente une viscosité à 25°C supérieure ou égale à 5 cSt et inférieure ou égale à 10 cSt.

9. Composition selon l'une quelconque des revendications 1 à 8, **caractérisée en ce qu'**elle comprend:
- une huile ester non volatile, notamment choisie dans le groupe comprenant le palmitate de 2-éthyl-hexyle, le palmitate d'isopropyle, le palmitate d'isooctyle, le palmitate d'isocétyle, le palmitate d'isodécyle, le palmitate d'isostéaryle, le palmitate de 2-octyl-décyle, le néopentanoate d'isodécyle, l'octanoate d'isocétyle, l'isononanoate d'isononyle, l'isononanoate d'isodécyle, l'isononanoate de tridécyle, le laurate d'hexyle, le laurate de 2-hexyl-décyle, le myristate d'isopropyle, le myristate d'isocétyle, le myristate d'isotridécyle, le myristate de 2-octyldodécyle, l'isostéarate d'isopropyle, le stéarate d'octyl-2 dodécyle, l'isostéarate d'isostéaryle, l'érucate d'octyl-2 dodécyle, le diisostéaryl malate et leurs mélanges; et optionnellement
- une huile non volatile choisie dans le groupe comprenant les huiles hydrocarbonées ; les (poly) esters synthétiques et (poly) éthers, en particulier les (poly) esters d'acides en C6-C20 et d'alcools en C6-C20, avantageusement ramifiés tels que l'isononanoate d'isononyle ; les huiles végétales ; les acides gras ramifiés et/ou insaturés ; les alcools gras ramifiés et/ou insaturés tels que l'octyldodécanol ; les huiles fluorosilicone ; les huiles fluorées ; et leurs mélanges.

10. Composition selon l'une quelconque des revendications 1 à 9, **caractérisée en ce que** l'agent structurant de la phase huileuse est :
- une cire polaire, à savoir une cire comprenant au moins un hétéroatome tel que l'oxygène, l'azote, le silicium ou le phosphore,
- une cire apolaire hydrocarbonée, à savoir une cire comprenant uniquement des atomes de carbone et d'hydrogène et ne comprenant pas d'hétéroatomes tels que l'oxygène, l'azote, le silicium ou le phosphore,
- une cire apolaire siliconée, à savoir une cire comprenant un hétéroatome de silicium, et/ou,
- un gélifiant huileux choisi dans le groupe comprenant un polymère siliconé ; un copolymère cyclique de vinyl diméthicone/diméthicone ; une résine polyamide ou une résine poly (ester-amide) ; un copolymère de styrène et d'au moins une oléfine autre que le styrène ; un diamide d'acide N-acyl glutamique ; un ester de dextrine ; un sucroester et leurs mélanges.

11. Composition selon l'une quelconque des revendications 1 à 10, **caractérisée en ce que** l'agent structurant de la phase huileuse est une cire choisie dans le groupe comprenant une cire de polyéthylène, seule ou en association avec une autre cire de polyéthylène, un mélange de cire de polyéthylène et de cire d'esters végétaux, un mélange de cire synthétique et de cire de carnauba, les esters de jojoba et leurs mélanges.

12. Composition selon l'une quelconque des revendications 1 à 11, **caractérisée en ce qu'**elle comprend :
- un mélange émulsionnant comprenant au moins un émulsionnant siliconé présent dans une quantité comprise entre 0,1% et 5% et au moins un silicone élastomère émulsionnant ledit silicone élastomère étant présent dans une quantité comprise entre 0,05% et 3%,
- au moins une huile non volatile présente dans une quantité comprise entre 30% et 50% et comprenant au moins une huile non volatile siliconée dans une quantité comprise entre 10% et 50% et une seconde huile choisie parmi au moins une huile ester non volatile,
- 2% à 12%, de préférence 6% à 10%, d'un agent structurant de la phase huileuse,
- 30% à 50% d'eau,
- 0% à 2% et de préférence 0% d'une huile siliconée volatile et/ou d'une huile hydrocarbonée volatile, et
- optionnellement un filmogène dans une quantité inférieure ou égale à 10%;
les pourcentages définis ci-dessus étant des pourcentages en poids par rapport au poids total de la composition.

13. Composition selon l'une quelconque des revendications 1 à 12, **caractérisée en ce qu'**elle comprend en outre :
- un émulsionnant non siliconé, notamment choisi dans le groupe comprenant les polymères du type ester d'acide gras de glycol polyoxyalkyléné, tels que ceux identifiés par le nom INCI Polysorbate 60, Polysorbate 20, Polysorbate 85, et leurs mélanges,
- un humectant choisi dans le groupe comprenant le glycérol ; les glycols, tels que le propylèneglycol, le butylèneglycol et l'hexylèneglycol ; les alcools de sucre ou les polyols de sucre, tels que le sorbitol, le xylitol et le maltitol ; les polyols polymères tels que le polydextrose ; les alpha-hydroxyacides tels que l'acide lactique ; l'acide hyaluronique ; l'urée ; l'acide L-pyrrolidone carboxylique (PCA) ; et leurs mélanges,
- une charge choisie dans le groupe comprenant le talc, le mica, la silice, le kaolin, le nitrure de bore, la lauroyl lysine, la séricite, les billes de cellulose, les billes de verre, l'amidon, l'amidon modifié par l'anhydride octénylsuccinique, les résines de silicone telles que le polyméthylsilsesquioxane disponible sous la dénomination Tospearl 2000B de Momentive Performance Materials, les poudres dérivées d'élastomères de silicone tels que l'élastomère de silicone revêtu avec une résine de silicone disponible sous la dénomination commerciale KSP100 ou KSP300 de ShinEtsu, et leurs mélanges
- une matière colorante choisie dans le groupe comprenant les colorants hydrosolubles, les colorants liposolubles, les particules ayant pour effet de colorer et/ou d'opacifier la composition, telles que les pigments, les nacres, les laques (colorants hydrosolubles adsorbés sur un support inorganique inerte), et leurs mélanges,
- un agent conservateur choisi dans le groupe comprenant le phénoxyéthanol, le sorbate de potassium, le déhydroacétate de sodium, la chlorphénésine, les parabènes tels que le méthylparabène ou le propylparabène, et leurs mélanges et/ou
- du chlorure de sodium.

14. Composition selon la revendication 13, **caractérisée en ce qu'**elle comprend :
- 0% à 5% d'émulsionnant non siliconé, de préférence 0,1% à 1%,
- 0% à 10% d'humectant, de préférence 1% à 6%,
- 0% à 25% de charge, de préférence 2% à 10%,
- 0% à 10% de matière colorante, de préférence 2% à 6%,
- 0% à 2% d'agent conservateur, de préférence 0,1% à 1%,
- 0% à 1,5% de chlorure de sodium, de préférence de 0,3 à 0,7%,
les pourcentages définis ci-dessus étant des pourcentages en poids par rapport au poids total de la composition.

15. Composition selon l'une quelconque des revendications 1 à 14, **caractérisée en ce qu'**elle présente une dureté comprise entre 70 g et 150 g (grammes), de préférence comprise entre 80 g et 120 g, et plus préférentiellement encore comprise entre 80 g et 100 g, ladite dureté étant déterminée par la mesure de la force de compression mesurée à 20°C à l'aide du texturomètre vendu sous la dénomination « TA-XT Plus Microstable System » par la société Swantech.

16. Composition selon l'une quelconque des revendications 1 à 15, **caractérisée en ce qu'**elle se présente sous la forme d'un stick, de préférence d'un stick de maquillage pour la peau ou les lèvres et/ou d'un stick de soin non thérapeutique pour la peau ou les lèvres.

17. Procédé cosmétique de maquillage et/ou de soin non thérapeutique de la peau ou des lèvres, **caractérisé par le fait qu'**il consiste à appliquer sur la peau ou les lèvres une composition telle que définie à l'une quelconque des revendications 1 à 16.

## Patentansprüche

1. Kosmetische Zusammensetzung in Form einer festen Wasser-in-ÖI-Emulsion, **dadurch gekennzeichnet, dass** sie umfasst:
- eine Emulgiermischung, umfassend mindestens einen Silikonemulgator, der in einer Menge zwischen 0,1% und 5% vorhanden ist, und mindestens einen elastomeren Silikonemulgator, wobei das elastomere Silikon in einer Menge zwischen 0,05% und 3% vorhanden ist;
- mindestens ein nichtflüchtiges Öl, das in einer Menge zwischen 30% und 50% vorhanden ist und mindestens ein nichtflüchtiges Silikonöl und gegebenenfalls ein zweites Öl umfasst, das aus mindestens einem nichtflüchtigen Esteröl ausgewählt ist;
- ein Strukturierungsmittel der öligen Phase, umfassend ein Wachs und/oder ein öliges Geliermittel,
- 30% bis 50% Wasser,
- 0% bis 2% und noch bevorzugter 0% eines flüchtigen Silikonöls und/oder eines flüchtigen Kohlenwasserstofföls und
- gegebenenfalls einen Filmbildner;
wobei die oben definierten Prozentsätze Gewichtsprozente in Bezug auf das Gesamtgewicht der Zusammensetzung sind.

2. Zusammensetzung nach Anspruch 1, **dadurch gekennzeichnet, dass** der Silikonemulgator ein Siloxanpolymer ist, umfassend:
- eine Fett-Seitenkette,
- eine oxyethylenierte oder oxypropylenierte Seitenkette und/oder eine polyethoxylierte Seitenkette und gegebenenfalls
- eine Silikonseitenkette.

3. Zusammensetzung nach Anspruch 1 oder Anspruch 2, **dadurch gekennzeichnet, dass** der Silikonemulgator ausgewählt ist aus der Gruppe umfassend:
- die Verbindung der Formel (I): 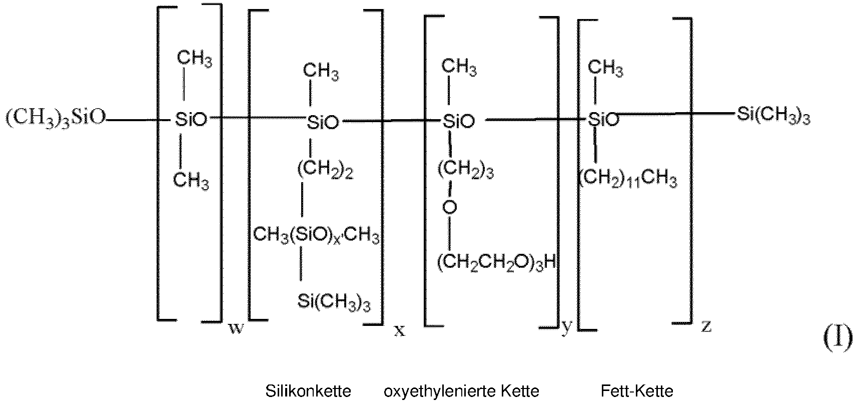 wobei w eine ganze Zahl zwischen 1 und 1000 ist, x' eine ganze Zahl zwischen 1 und 50 ist, x, y und z unabhängig voneinander eine ganze Zahl zwischen 1 und 100 darstellen,
- die Verbindung der Formel (II): 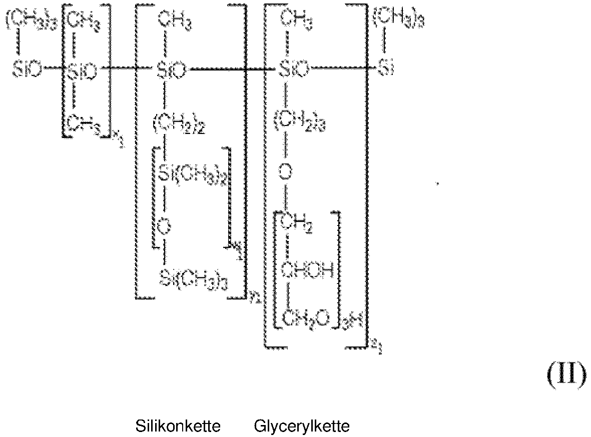 wobei x₁ eine ganze Zahl zwischen 1 und 1000 ist, w₁ eine ganze Zahl zwischen 1 und 50 ist und y₁ und z₁ unabhängig voneinander eine ganze Zahl zwischen 1 und 100 darstellen,
- die Verbindung der Formel (III): 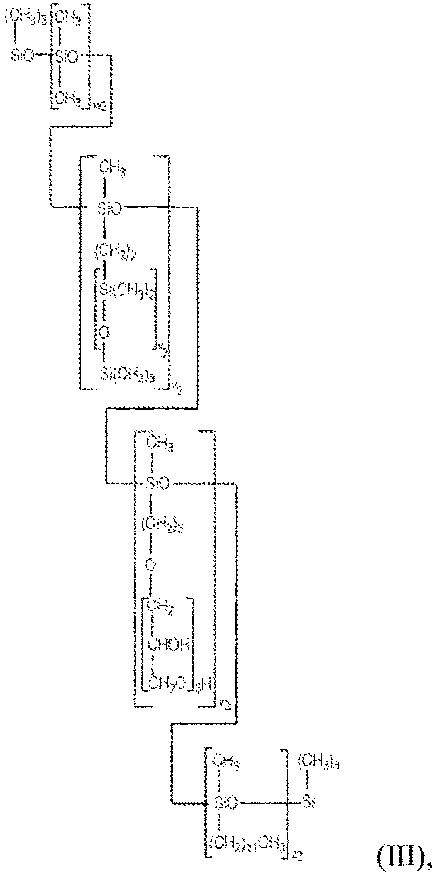 wobei w₂ eine ganze Zahl zwischen 1 und 1000 ist, v₂ eine ganze Zahl zwischen 1 und 50 ist, x_{2,} y₂ und z₂ unabhängig voneinander eine ganze Zahl zwischen 1 und 100 darstellen, und Mischungen davon.

4. Zusammensetzung nach einem der Ansprüche 1 bis 3, **dadurch gekennzeichnet, dass** sie einen elastomeren Silikonemulgator umfasst, ausgewählt aus vernetzten elastomeren Organopolysiloxanen, vorzugsweise mit einer polyhydroxylierten Kette.

5. Zusammensetzung nach einem der Ansprüche 1 bis 4, **dadurch gekennzeichnet, dass** das nichtflüchtige Silikonöl ausgewählt ist aus der Gruppe umfassend die Polydimethylsiloxane (PDMS) mit mindestens 8 Siliziumatomen, die Polyalkylmethylsiloxane, deren Alkylkette 8 bis 20 Kohlenstoffatome aufweist, die Alkyldimethicone, die Vinylmethylmethicone, die Öle, die unter dem INCI-Namen Phenyltrimethicone identifiziert sind, und Mischungen davon.

6. Zusammensetzung nach einem der Ansprüche 1 bis 5, **dadurch gekennzeichnet, dass** der Filmbildner ausgewählt ist aus Silikonharzen und insbesondere den Trimethylsiloxysilikaten, den Silikonacrylaten und insbesondere den Acrylaten/Polytrimethylsiloxymethacrylat-Copolymer, den Polybutenen oder den Harzen pflanzlichen Ursprungs wie Shorea Robusta Harz oder Mischungen davon.

7. Zusammensetzung nach einem der Ansprüche 1 bis 6, **dadurch gekennzeichnet, dass** die Menge an nichtflüchtigem Silikonöl zwischen 10% und 50% liegt und die Menge an Filmbildner, wenn er vorhanden ist, zwischen 1% und 15% liegt; vorzugsweise zwischen 1% und 10%, wobei die oben definierten Prozentsätze Gewichtsprozente bezogen auf das Gesamtgewicht der Zusammensetzung sind.

8. Kosmetische Zusammensetzung nach einem der Ansprüche 1 bis 7, **dadurch gekennzeichnet, dass** das nichtflüchtige Silikonöl bei 25 °C eine Viskosität von mehr als oder gleich 5 cSt und kleiner oder gleich 10 cSt aufweist.

9. Zusammensetzung nach einem der Ansprüche 1 bis 8, **dadurch gekennzeichnet, dass** sie umfasst:
- ein nichtflüchtiges Esteröl, insbesondere ausgewählt aus der Gruppe umfassend 2-Ethylhexylpalmitat, Isopropylpalmitat, Isooctylpalmitat, Isocetylpalmitat, Isodecylpalmitat, Isostearylpalmitat, 2-Octyldecylpalmitat, Isodecylneopentanoat, Isocetyloctanoat, Isononylisononanoat, Isodecylisononanoat, Tridecylisononanoat, Hexyllaurat, 2-Hexyldecyllaurat, Isopropylmyristat, Isocetylmyristat, Isotridecylmyristat, 2-Octyldodecylmyristat, Isopropylisostearat, Octyl-2-dodecylstearat, Isostearylisostearat, Octyl-2-dodecylerukat, Diisostearylmalat und Mischungen davon; und gegebenenfalls
- ein nichtflüchtiges Öl, ausgewählt aus der Gruppe umfassend die Kohlenwasserstofföle; die synthetischen (Poly)ester und (Poly)ether, insbesondere die (Poly)ester von C6-C20-Säuren und C6-C20-Alkoholen, vorteilhafterweise verzweigt, wie Isononylisononanoat; die Pflanzenöle; die verzweigten und/oder ungesättigten Fettsäuren; die verzweigten und/oder ungesättigten Fettalkohole wie Octyldodecanol; die Fluorsilikonöle; die fluorierten Öle; und Mischungen davon.

10. Zusammensetzung nach einem der Ansprüche 1 bis 9, **dadurch gekennzeichnet, dass** das Strukturierungsmittel der öligen Phase ist:
- ein polares Wachs, nämlich ein Wachs, umfassend mindestens ein Heteroatom wie Sauerstoff, Stickstoff, Silizium oder Phosphor,
- ein unpolares Kohlenwasserstoffwachs, nämlich ein Wachs, das nur Kohlenstoff- und Wasserstoffatome umfasst und keine Heteroatome wie Sauerstoff, Stickstoff, Silizium oder Phosphor umfasst;
- ein unpolares Silikonwachs, nämlich ein Wachs, das ein Siliziumheteroatom umfasst, und/oder
- ein öliges Geliermittel, ausgewählt aus der Gruppe, umfassend ein Silikonpolymer; ein cyclisches Vinyldimethicon/Dimethicon-Copolymer; ein Polyamidharz oder ein Poly(ester-amid)harz; ein Copolymer aus Styrol und mindestens einem anderen Olefin als Styrol; ein N-Acylglutaminsäurediamid; ein Dextrinester; ein Sucroester und Mischungen davon.

11. Zusammensetzung nach einem der Ansprüche 1 bis 10, **dadurch gekennzeichnet, dass** das Strukturierungsmittel der öligen Phase ein Wachs ist, das ausgewählt ist aus der Gruppe umfassend ein Polyethylenwachs, allein oder in Kombination mit einem anderen Polyethylenwachs, einer Mischung aus Polyethylenwachs und Pflanzenesterwachs, eine Mischung aus synthetischem Wachs und Carnaubawachs, die Jojobaester und Mischungen davon.

12. Zusammensetzung nach einem der Ansprüche 1 bis 11, **dadurch gekennzeichnet, dass** sie umfasst:
- eine Emulgiermischung, umfassend mindestens einen Silikonemulgator, der in einer Menge zwischen 0,1% und 5% vorhanden ist, und mindestens einen elastomeren Silikonemulgator, wobei das elastomere Silikon in einer Menge zwischen 0,05% und 3% vorhanden ist;
- mindestens ein nichtflüchtiges Öl, das in einer Menge zwischen 30% und 50% vorhanden ist und mindestens ein nichtflüchtiges Silikonöl in einer Menge zwischen 10% und 50% und ein zweites Öl, das aus mindestens einem nichtflüchtigen Esteröl ausgewählt ist, umfasst;
- 2% bis 12%, vorzugsweise 6% bis 10% eines Strukturierungsmittels der öligen Phase,
- 30% bis 50% Wasser,
- 0% bis 2% und bevorzugt 0% eines flüchtigen Silikonöls und/oder eines flüchtigen Kohlenwasserstofföls und
- gegebenenfalls einen Filmbildner in einer Menge von weniger oder gleich 10%;
wobei die oben definierten Prozentsätze Gewichtsprozente in Bezug auf das Gesamtgewicht der Zusammensetzung sind.

13. Zusammensetzung nach einem der Ansprüche 1 bis 12, **dadurch gekennzeichnet, dass** sie ferner umfasst:
- einen Nicht-Silikon-Emulgator, insbesondere ausgewählt aus der Gruppe, umfassend die Polymere vom Typ Fettsäureester von polyoxyalkyleniertem Glykol, wie diejenigen, die durch die INCI-Namen Polysorbat 60, Polysorbat 20, Polysorbat 85 identifiziert sind und Mischungen davon,
- ein Feuchthaltemittel, ausgewählt aus der Gruppe, umfassend Glycerin; die Glykole wie Propylenglykol, Butylenglykol und Hexylenglykol; die Zuckeralkohole oder die Zuckerpolyole wie Sorbitol, Xylit und Maltitol; die Polymerpolyole wie Polydextrose; die Alpha-Hydroxysäuren wie Milchsäure; Hyaluronsäure; Harnstoff; L-Pyrrolidoncarbonsäure (PCA); und Mischungen davon,
- einen Füllstoff, ausgewählt aus der Gruppe umfassend Talk, Glimmer, Kieselsäure, Kaolin, Bornitrid, Lauroyllysin, Sericit, Celluloseperlen, Glasperlen, Stärke, Stärke modifiziert durch Octenylbernsteinsäureanhydrid, Silikonharze wie Polymethylsilsesquioxan, erhältlich unter dem Namen Tospearl 2000B von Momentive Performance Materials, die Pulver, die von Silikonelastomeren abgeleitet sind wie dem Silikonelastomer, das mit einem Silikonharz beschichtet ist, das unter dem kommerziellen Namen KSP100 oder KSP300 von ShinEtsu erhältlich ist, und Mischungen davon
- ein Farbstoff-Material, ausgewählt aus der Gruppe umfassend wasserlösliche Farbstoffe, fettlösliche Farbstoffe, Partikel, die die Zusammensetzung färben und/oder trüben, wie Pigmente, Perlmutt, Lacke (wasserlösliche Farbstoffe, die auf einem inerten anorganischen Träger adsorbiert sind) und Mischungen davon,
- ein Konservierungsmittel, ausgewählt aus der Gruppe umfassend Phenoxyethanol, Kaliumsorbat, Natriumdehydroacetat, Chlorphenesin, Parabene wie Methylparaben oder Propylparaben und Mischungen davon, und/oder
- Natriumchlorid.

14. Zusammensetzung nach Anspruch 13, **dadurch gekennzeichnet, dass** sie umfasst:
- 0% bis 5% Nicht-Silikon-Emulgator, vorzugsweise 0,1% bis 1%,
- 0% bis 10% Feuchthaltemittel, vorzugsweise 1% bis 6%,
- 0% bis 25% Füllstoff, vorzugsweise 2% bis 10%,
- 0% bis 10% Farbstoff-Material, vorzugsweise 2% bis 6%,
- 0% bis 2% Konservierungsmittel, vorzugsweise 0,1% bis 1%,
- 0% bis 1,5% Natriumchlorid, vorzugsweise 0,3 bis 0,7%,
wobei die oben definierten Prozentsätze Gewichtsprozente in Bezug auf das Gesamtgewicht der Zusammensetzung sind.

15. Zusammensetzung nach einem der Ansprüche 1 bis 14, **dadurch gekennzeichnet, dass** sie eine Härte zwischen 70 g und 150 g (Gramm), vorzugsweise zwischen 80 g und 120 g und bevorzugter noch zwischen 80 g und 100 g aufweist, wobei die Härte durch Messen der bei 20 °C gemessenen Druckkraft unter Verwendung des von der Firma Swantech unter dem Namen "TA-XT Plus Microstable System" vertriebenen Texturometers bestimmt ist.

16. Zusammensetzung nach einem der Ansprüche 1 bis 15, **dadurch gekennzeichnet, dass** sie in Form eines Stifts vorliegt, vorzugsweise eines Make-up-Stifts für die Haut oder die Lippen und/oder eines nichttherapeutischen Pflege-Stifts für die Haut oder die Lippen.

17. Kosmetisches Verfahren zum Schminken und/oder zur nichttherapeutischen Pflege der Haut oder der Lippen, **dadurch gekennzeichnet, dass** es darin besteht, auf die Haut oder die Lippen eine Zusammensetzung aufzutragen, wie sie in einem der Ansprüche 1 bis 16 definiert ist.

## Claims

1. A cosmetic composition in the form of a solid water-in-oil emulsion **characterised in that** it comprises:
- an emulsifying mixture comprising at least one silicone emulsifier present in an amount between 0.1% and 5% and at least one emulsifying silicone elastomer said silicone elastomer being present in an amount between 0.05% and 3%,
- at least one non-volatile oil present in an amount between 30% and 50% and comprising at least one non-volatile silicone oil and optionally a second oil selected from at least one non-volatile ester oil,
- a structuring agent of the oily phase comprising a wax and/or an oily gelling agent,
- 30% to 50% water,
- 0% to 2%, and still more preferably 0% of a volatile silicone oil and/or a volatile hydrocarbon oil, and
- optionally a film-forming agent;
the percentages defined above being percentages by weight relative to the total weight of the composition.

2. A composition according to claim 1, **characterised in that** the silicone emulsifier is a siloxane polymer comprising:
- a fatty side chain,
- an oxyethylenated or oxypropylenated side chain and/or a polyethoxylated side chain, and optionally
- a silicone side chain.

3. A composition according to claim 1 or claim 2, **characterised in that** the silicone emulsifier is selected from the group comprising:
- the compound of formula (I): 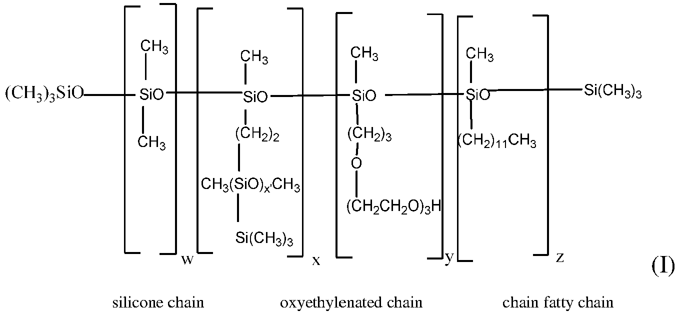 wherein w is an integer from 1 to 1,000, x is an integer between 1 and 50, x, y and z independently of one another represent an integer between 1 and 100,
- the compound of formula (II): 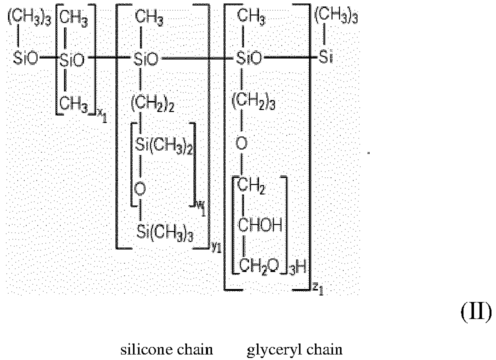 wherein x is an integer between 1 and 1000, w₁ is an integer between 1 and 50, y₁ and z₁ independently of one another represent an integer between 1 and 100,
- the compound of formula (III): 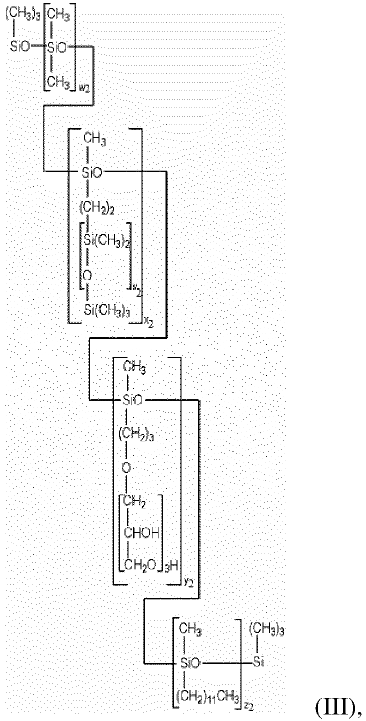 wherein w₂ is an integer between 1 and 1000, v₂ is an integer between 1 and 50, x₂, y₂ and z₂ independently from each other represent an integer between 1 and 100,
and mixtures thereof.

4. A composition according to any one of claims 1 to 3, **characterised in that** it comprises an emulsifying silicone elastomer chosen from crosslinked elastomeric organopolysiloxanes, preferably with a polyhydroxylated chain.

5. A composition according to any one of claims 1 to 4, **characterised in that** the non-volatile silicone oil is chosen from the group comprising polydimethylsiloxanes (PDMS) containing at least 8 silicon atoms, polyalkylmethylsiloxanes whose alkyl chain contains from 8 to 20 carbon atoms, alkyl dimethicones, vinyl methyl methicones, oils identified by the INCI name phenyltrimethicones and mixtures thereof.

6. A composition according to any one of claims 1 to 5, **characterised in that** the film-forming agent is selected from silicone resins and especially trimethylsiloxysilicates, acrylates and silicones especially Acrylates/Polytrimethylsiloxymethacrylate Copolymer, polybutenes or plant-derived resins such as Shorea robusta resin or mixtures thereof.

7. A composition according to any one of claims 1 to 6 **characterised in that** the amount of non-volatile silicone oil is between 10% and 50%, and the amount of film-forming agent when present is between 1% and 15 %, preferably between 1% and 10%, the percentages defined above being percentages by weight relative to the total weight of the composition.

8. The cosmetic composition of any one of claims 1 to 7 **characterised in that** the non-volatile silicone oil has a viscosity at 25°C greater than or equal to 5 cSt and less than or equal to 10 cSt.

9. A composition according to any one of claims 1 to 8, **characterised in that** it comprises:
- a non-volatile ester oil, in particular selected from the group comprising the palmitate, 2-ethylhexyl, isopropyl palmitate, isooctyl palmitate, isocetyl palmitate, isodecyl palmitate isostearyl palmitate, 2-octyl-decyl, isodecyl neopentanoate, isocetyl octanoate, isononyl isononanoate, isodecyl isononanoate, tridecyl isononanoate, hexyl laurate, 2-hexyl-decyl laurate, isopropyl myristate, isocetyl myristate, isotridecyl myristate, 2-octyldodecyl myristate, isopropyl isostearate, octyl-2 dodecyl stearate, isostearyl isostearate, 2-octyldodecyl erucate, diisostearyl malate and mixtures thereof; and optionally
- a non-volatile oil selected from the group comprising hydrocarbon oils; (poly) esters and synthetic (poly) ethers, in particular C6-C20 acid (poly) esters and C6-C20 alcohols, preferably branched such as isononyl isononanoate; vegetable oils; branched and/or unsaturated fatty acids; branched fatty alcohols and/or unsaturated such as octyldodecanol; fluorosilicone oils; fluorinated oils; and mixtures thereof.

10. A composition according to any one of claims 1 to 9, **characterised in that** the structuring agent of the oily phase is:
- a polar wax, i.e. a wax comprising at least one heteroatom such as oxygen, nitrogen, silicon or phosphorus,
- a hydrocarbon apolar wax, i.e. a wax containing only carbon atoms and hydrogen and containing no hetero atoms such as oxygen, nitrogen, silicon or phosphorus,
- a silicone apolar wax, i.e. a wax comprising a silicon heteroatom, and/or,
- an oily gelling agent chosen from the group comprising a silicone polymer; a cyclic copolymer of vinyl dimethicone/dimethicone; a polyamide resin or a resin poly (ester-amide); a copolymer of styrene and at least one olefin other than styrene; an N-acyl glutamic acid diamide; a dextrin ester; a sucrose ester and mixtures thereof.

11. A composition according to any one of claims 1 to 10, **characterised in that** the structuring agent of the oily phase is a wax selected from the group consisting of polyethylene wax, alone or in combination with another polyethylene wax, a mixture of polyethylene wax and of vegetable ester wax, a mixture of synthetic wax and carnauba wax, jojoba esters, and mixtures thereof.

12. A composition according to any one of claims 1 to 11, **characterised in that** it comprises:
- an emulsifying mixture comprising at least one silicone emulsifier present in an amount between 0.1% and 5% and at least one emulsifying elastomer silicone said elastomeric silicone being present in an amount between 0.05% and 3%,
- at least one non-volatile oil present in an amount between 30% and 50% and comprising at least one non-volatile silicone oil in an amount between 10% and 50% and a second oil selected from at least one non-volatile ester oil,
- 2% to 12%, preferably 6% to 10%, of a structuring agent for the oily phase,
- 30% to 50% water,
- 0% to 2% and preferably 0% of a volatile silicone oil and/or a volatile hydrocarbon oil, and
- optionally a film-forming agent in an amount less than or equal to 10%;
the percentages defined hereinbefore being percentages by weight relative to the total weight of the composition.

13. A composition according to any one of claims 1 to 12, **characterised in that** it further comprises:
- a non-silicone emulsifier, chosen in particular from the group comprising polymers of the glycol fatty acid ester polyoxyalkylene type, such as those identified by the INCI name Polysorbate 60, Polysorbate 20, Polysorbate 85 and mixtures thereof,
- a humectant selected from the group consisting of glycerol; glycols, such as propylene glycol, butylene glycol and hexylene glycol; sugar alcohols or sugar polyols such as sorbitol, xylitol and maltitol; polymer polyols such as polydextrose; alpha-hydroxy acids such as lactic acid; hyaluronic acid; urea; L-pyrrolidone carboxylic acid (PCA); and mixtures thereof,
- an additive elected from the group consisting of talc, mica, silica, kaolin, boron nitride, lauroyl lysine, sericite, cellulose beads, glass beads, starch, starch modified by octenyl succinic anhydride, silicone resins such as polymethylsilsesquioxane available under the Momentive Performance Materials tradename Tospearl 2000B, powders derived from silicone elastomers such as silicone elastomer coated with a silicone resin available under the ShinEtsu tradenames KSP 100 or KSP300 of, and mixtures thereof
- a colorant selected from the group comprising water-soluble dyes, liposoluble dyes, particles having the effect of colouring and/or opacifying the composition, such as pigments, pearlescent agents, lacquers (water-soluble dyes adsorbed onto a support inert inorganic), and mixtures thereof,
- a preservative selected from the group consisting of phenoxyethanol, potassium sorbate, sodium dehydroacetate, chlorphenesin, parabens such as methylparaben or propylparaben, and mixtures thereof and/or
- sodium chloride.

14. A composition according to claim 13, **characterised in that** it comprises:
- 0% to 5% of a non-silicone emulsifier, preferably 0.1% to 1%,
- 0% to 10% of a humectant, preferably 1% to 6%,
- 0% to 25% an additive, preferably 2% to 10%,
- 0% to 10% of a colorant, preferably 2% to 6%,
- 0% to 2% of a preservative, preferably 0.1% to 1%,
- 0% to 1.5% sodium chloride, preferably 0.3 to 0.7%,
the percentages defined above being percentages by weight relative to the total weight of the composition.

15. A composition according to any one of claims 1 to 14, **characterised in that** it has a hardness of between 70 g and 150 g (grams), preferably between 80 g and 120 g, and even more preferably between 80 g and 100 g, said hardness being determined by measuring the compression strength measured at 20°C using a texturometer marketed by Swantech under the name "TA-XT Plus Microstable System".

16. A composition according to any one of claims 1 to 15, **characterised in that** it is in the form of a stick, preferably a make-up stick for the skin or the lips and/or non-therapeutic care stick for the skin or lips.

17. A cosmetic method for making up and/or for the nontherapeutic care of the skin or the lips, **characterised in that** it consists in applying a composition as defined in any one of claims 1 to 16 to the skin or the lips.
